(19) **Europäisches Patentamt**
**European Patent Office**
**Office européen des brevets**

(11) **EP 3 734 259 B1**

(12) **EUROPÄISCHE PATENTSCHRIFT**

(45) Veröffentlichungstag und Bekanntmachung des Hinweises auf die Patenterteilung:
**15.01.2025 Patentblatt 2025/03**

(21) Anmeldenummer: **20171888.9**

(22) Anmeldetag: **28.04.2020**

(51) Internationale Patentklassifikation (IPC):
**G01N 23/083** (2018.01)    **G01N 33/02** (2006.01)
**G01N 23/18** (2018.01)    **G01N 23/087** (2018.01)

(52) Gemeinsame Patentklassifikation (CPC):
**G01N 23/087; G01N 23/18; G01N 33/02;**
G01N 2223/04; G01N 2223/1016; G01N 2223/3308;
G01N 2223/5015; G01N 2223/618; G01N 2223/633;
G01N 2223/643; G01N 2223/652

(54) **RÖNTGENSTRAHLUNGSDETEKTORVORRICHTUNG UND VORRICHTUNG ZUR RÖNTGENINSPEKTION VON PRODUKTEN, INSBESONDERE VON LEBENSMITTELN**

X-RAY DETECTOR APPARATUS AND APPARATUS FOR X-RAY INSPECTION OF PRODUCTS, IN PARTICULAR FOOD PRODUCTS

DISPOSITIF DE DÉTECTION DU RAYONNEMENT X ET DISPOSITIF D'INSPECTION PAR RAYONS X DES PRODUITS, EN PARTICULIER DES PRODUITS ALIMENTAIRES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **03.05.2019 DE 102019111463**

(43) Veröffentlichungstag der Anmeldung:
**04.11.2020 Patentblatt 2020/45**

(73) Patentinhaber: **WIPOTEC GmbH**
**67657 Kaiserslautern (DE)**

(72) Erfinder:
• **Siegrist, Michael**
 **67659 Kaiserslautern (DE)**
• **Heil, Ulrich**
 **66907 Glan-Münchweiler (DE)**
• **Bur, Christian**
 **66113 Saarbrücken (DE)**
• **Hoffmann, Kai**
 **67475 Weidenthal (DE)**

(74) Vertreter: **Eder Schieschke & Partner mbB**
**Patentanwälte**
**Elisabethstraße 34**
**80796 München (DE)**

(56) Entgegenhaltungen:
EP-A1- 3 287 774    WO-A1-2017/205914
WO-A1-2018/102051    US-A1- 2010 012 845
US-A1- 2013 079 918

**Beschreibung**

Die Erfindung betrifft eine Vorrichtung zur Röntgeninspektion von Produkten, insbesondere von Lebensmitteln.

Zur Röntgeninspektion von bewegten Produkten finden üblicherweise Zeilendetektoren Verwendung, die quer zur Bewegungsrichtung der zu untersuchenden Produkte vorgesehen sind. Anstelle einer Bewegung des Produkts kann auch die gesamte Vorrichtung zur Röntgeninspektion oder zumindest die betreffende Röntgenstrahlungsdetektorvorrichtung relativ zu dem zu untersuchenden Produkt bewegt werden. Das zu untersuchende Produkt wird mittels des Zeilendetektors, welcher die von einer oder mehreren Röntgenstrahlungsquellen erzeugte Röntgenstrahlung detektiert, gescannt, und die zeilenweise erzeugten Bilddaten werden in ein Bild des zu untersuchenden Produkts umgesetzt. Zur Erzeugung des Bildes können di Bilddaten in geeigneter Weise verarbeitet werden. Das so erzeugte Bild kann durch Bildverarbeitung inspiziert werden. Insbesondere kann das Bild des zu untersuchenden Produkts daraufhin inspiziert werden, ob ein oder mehrere vorgegebene Merkmale vorliegen bzw. erfüllt sind. Beispielsweise kann ein Lebensmittel, beispielsweise ein Fleischstück, daraufhin untersucht werden, ob sich darin unerwünschte Fremdkörper befinden, wie Knochensplitter, Metallspäne von Verarbeitungsmaschinen, Glassplitter, Kunststoffe, Steine oder dergleichen.

Hierzu ist es bekannt, spektral integrierende Zeilendetektoren (Nicht-Spektraldetektoren) zu verwenden, die praktisch die gesamte Breite des Röntgenstrahlenspektrums der Röntgenstrahlung detektieren, die von der betreffenden Röntgenstrahlungsquelle erzeugt wird. Derartige Zeilendetektoren besitzen eine vergleichsweise hohe Ortsauflösung im Bereich von beispielsweise 0,2 mm über eine Gesamt-Detektorbreite von beispielsweise 200 bis 800 mm oder mehr. Die Zeilendetektoren können dabei in Form von Modulen aufgebaut sein, die sich mit jeweils nur sehr geringen Lücken von nur wenigen Pixeln (beispielsweise ein bis zwei Pixel pro Modulrand) bis zum Erreichen einer gewünschten Scan-Breite koppeln lassen. Mit derartigen Nicht-Spektraldetektoren sind somit auch sehr kleine Fremdkörper bzw. Kontaminationen detektierbar. Dieser Detektortyp benötigt zudem keine Kühlung und ist kostengünstig herstellbar.

Allerdings erzeugen derartige Nicht-Spektraldetektoren infolge der spektralen Integration lediglich Grauwerte. Ein Grauwert ist dabei abhängig von der Dämpfung der Röntgenstrahlung beim Hindurchtreten durch das zu untersuchende Produkt. Die Dämpfung ist dabei wiederum abhängig von der Dicke des Produkts und den Materialeigenschaften.

Dieser Sensortyp eignet sich insbesondere für das Detektieren von kleinsten, stark absorbierenden Fremdkörpern, beispielsweise von Metallsplittern.

Eine Verbesserung des Kontrastes des durch Zeilendetektoren erzeugten Bildes kann durch das Dual-Energy-Verfahren erreicht werden. Dabei werden zwei Zeilendetektoren verwendet, deren Scan-Bilder überlagert werden. Die Zeilendetektoren erfassen dabei unterschiedliche spektrale Bereiche der durch das Produkt hindurchgetretenen Röntgenstrahlung. Dies wird durch das Verwenden wenigstens eines Röntgenstrahlungsfilters erreicht, der im Strahlengang vor einem der Nicht-Spektraldetektoren angeordnet ist. Solche Filter arbeiten jedoch nur als Hochpassfilter und können zudem nicht ausreichend flexibel hinsichtlich der gewünschten Filterkante hergestellt werden. Zusätzlich dämpfen sie auch die zu detektierende Röntgenstrahlung im gewünschten Spektralbereich. Durch das getrennte Erfassen unterschiedlicher Spektralbereiche ist in den Bildsignalen der jeweiligen Zeilendirektoren eine unterschiedliche Information enthalten. Durch eine gewichtete Überlagerung (beispielsweise vorzeichenrichtige Addition der gewichteten Bilddaten) lässt sich ein Gesamtbild erzeugen, welches hinsichtlich der Erkennbarkeit bestimmter Fremdkörpermaterialien einen besseren Kontrast aufweist als ein Single-Energy-Bild. Mit einem festen Röntgenstrahlungsfilter lässt sich der Kontrast jedoch nur für ein oder mehrere bestimmte Materialien verbessern. Daher sind Dual-Energy-Verfahren hinsichtlich ihres Einsatzes wenig flexibel, da das Röntgenstrahlungsfilter, abhängig vom Anwendungsfall, in geeigneter Weise gewählt werden muss.

Zudem überlappen sich meist die Spektralbereiche der Röntgenstrahlung, die von den beiden Zeilendetektoren erfasst werden, so dass in jedem der beiden Bildsignale ein Teil derselben Information enthalten ist. Damit ist keine optimale Kontrastverbesserung erreichbar.

Das Dual-Energy-Verfahren ermöglicht jedoch das Ausblenden von Produktbereichen eines aus lediglich zwei Materialien bestehenden Produkts bei einer geeigneten Kombination der beiden Bildsignale. So kann beispielsweise der Kontrast im Bereich eines Fremdkörpers, d. h. eines ersten Materials, innerhalb des Produkts, d. h. eines zweiten Materials, optimiert werden. Dies gilt jedoch nur für ein im Wesentlichen homogenes Produkt aus einem einzigen Material (oder einer Kombination von Materialien mit sehr ähnlichen Dämpfungseigenschaften für die Röntgenstrahlung), in welchem Fremdkörper aus einem weiteren Material (mit unterschiedlichen Dämpfungseigenschaften für die Röntgenstrahlung) enthalten sind.

Weiterhin wurden in den letzten Jahren spektral auflösende Zeilendetektoren entwickelt, die ebenfalls modulartig gekoppelt werden können. Derartige Spektral-Zeilendetektoren, die ausreichend große Scan-Breiten von 200 bis 800 mm oder mehr ermöglichen, sind derzeit jedoch nur mit einer relativ groben Ortsauflösung, d. h. mit einer verhältnismäßig großen Pixel-Pitch, beispielsweise von 0,8 mm, verfügbar. Derartige Spektral-Zeilendetektoren sind in der Lage, die gesamte spektrale Breite der zu detektierenden Röntgenstrahlung zu erfassen, beispielsweise im Bereich von 20 keV bis 160 keV. Diese Detektoren stellen zur spektralen Auflösung eine Vielzahl, beispielsweise bis zu 256, Energiekanäle bereit. Ein derartiger Spektral-Zeilendetektor ermöglicht es daher, eine der Anzahl von Energiekanälen entsprechende Anzahl von Teilbildern zu erzeugen. Die in den Teilbildern jeweils enthaltene Information kann in vielfältiger Weise verwendet und verarbeitet werden. Insbesondere ermöglicht diese Information das Erkennen von Materialien bzw. Materialkombinationen sowie das Detektieren von Schichtdicken.

Neben dem Nachteil einer verhältnismäßig groben Ortsauflösung sind derartige Spektral-Zeilendetektoren teuer und weisen eine größere Baugröße auf als einfache nicht spektral auflösende Zeilendetektoren. Zudem wird zum Betrieb von Spektral-Zeilendetektoren deutlich mehr Energie benötigt als für den Betrieb von Nicht-Spektraldetektoren, wobei der hohe Energieverbrauch eine aufwändige Kühlung notwendig macht.

Ein weiterer wesentlicher Nachteil der Spektral-Zeilendetektoren ist die lange Anlaufzeit, die im Bereich von 30 Minuten liegen kann. Diese lange Anlaufzeit ist dadurch bedingt, dass der Spektral-Zeilendetektor erst nach einer gewissen Zeit eine ausreichende Ladungstrennung gewährleistet.

Weiterhin ist aus der US 2013/0079918 A1 ein Sortiersystem bekannt, mit dem Materialen, wie insbesondere Metalle, sortiert werden können. Das System arbeitet mit zwei Detektoren, wobei ein Detektor die zu sortierenden Materialen durchstrahlende Röntgenstrahlung und ein weiterer Detektor Fluoreszenzstrahlung, welche von den Materialien vor allem in der Nähe der Oberfläche erzeugt und in alle Richtungen abgegeben wird, erfasst. Der Fluoreszenzdetektor ist so angeordnet, dass er den Teil der Fluoreszenzstrahlung erfasst, der bei einer Bestrahlung der Materialien im Wesentlichen senkrecht von oben in einer Richtung schräg nach oben abgegeben wird. Sowohl der Fluoreszenzdetektor als auch der Durchlichtdetektor sind als spektral auflösende Sensoren ausgebildet, wobei die jeweils gegebene Ortsauflösung und spektrale Auflösung verschieden sein kann. Weiterhin ist aus WO 2017/205914 A1 eine Vorrichtung zur Röntgeninspektion von Produkten bekannt, die einen spektral auflösenden Zeilendetektor aufweist. Die gemessene Spektren werden beide als Energiedaten und, nach einer Integration, als Grauwerten verwendet. Die Ortsauflösung ist daher identisch für die Energiedaten und für die integrierte Grauwerte.

Ausgehend von diesem Stand der Technik liegt der Erfindung die Aufgabe zugrunde, eine Vorrichtung zur Röntgeninspektion von Produkten, insbesondere von Lebensmitteln, zu schaffen, die kostengünstig herstellbar ist und eine verbesserte Erkennung von Fremdkörpern in einem zu untersuchenden Produkt ermöglicht.

Die Erfindung löst diese Aufgabe mit den Merkmalen des Patentanspruchs 1. Weitere Ausführungsformen ergeben sich aus den Unteransprüchen.

Die Erfindung geht von der Erkenntnis aus, dass sich wenigstens ein nicht spektral auflösender Zeilendetektor mit höherer Ortsauflösung und ein spektral auflösender Zeilendetektor mit niedrigerer oder gleicher Ortsauflösung vorteilhaft kombinieren lassen, um eine hohe und Erkennungsgenauigkeit bei der höheren Ortsauflösung zu erreichen.

Die erfindungsgemäße Vorrichtung zur Röntgeninspektion weist eine Röntgenstrahlungsdetektorvorrichtung, eine Auswerte- und Steuereinheit und eine Strahlungserzeugungsvorrichtung mit mindestens einer Röntgenstrahlungsquelle zur Erzeugung von Röntgenstrahlung mit einer vorgegebenen spektralen Breite auf, wobei die Röntgenstrahlung ein in einer Bewegungsrichtung mit einer vorgegebenen Bewegungsgeschwindigkeit bewegtes zu untersuchendes Produkt durchstrahlt. Die Röntgenstrahlungsdetektorvorrichtung weist wenigstens einen ersten Zeilendetektor mit einer ersten diskreteren Ortsauflösung (d. h. einer größeren Anzahl von Pixeln pro Längeneinheit) auf, welcher zur Erfassung der breitbandigen Röntgenstrahlung entlang einer oder mehrerer paralleler erster Erfassungslinien quer zu einer Bewegungsrichtung einer Relativbewegung zwischen einem zu untersuchenden Produkt und der Röntgenstrahlungsdetektorvorrichtung ausgebildet ist und welcher die durch das zu untersuchende Produkt hindurchgetretene Röntgenstrahlung über eine vorgegebene Erfassungsbreite nicht-spektral aufgelöst detektiert und erste Bilddaten erzeugt, wobei die ersten Bilddaten einen Grauwert für jeden diskreten Ort umfassen.

An dieser Stelle sei erwähnt, dass der Begriff "Röntgenstrahlung" in der vorliegenden Beschreibung jegliche Art von hochenergetischer elektromagnetischer Strahlung umfasst, insbesondere Röntgenstrahlung im Bereich oberhalb von 100 eV, insbesondere oberhalb von 1 keV, sowie Terahertzstrahlung, d.h. Strahlung im Bereich von ca. 0,3 THz bis 30 THz.

Anstelle eines einzigen derartigen nicht spektral auflösenden Zeilendetektors mit höherer Ortsauflösung, d.h. einer geringen Pixel-Pitch, können auch zwei oder mehrere derartige Zeilendetektoren verwendet sein, die in an sich bekannter Weise zur Erzeugung eines Dual-Energy-Bildes ausgebildet sind, wobei zumindest einer dieser Zeilendetektoren ein Filter zur Filterung der zu detektierenden Röntgenstrahlung aufweist. Statt eines Filters kann in einer entsprechenden Vorrichtung zur Röntgeninspektion mit einer derartigen Röntgenstrahlungsdetektorvorrichtung auch eine weitere Röntgenstrahlungsquelle verwendet werden, die gegenüber einer ersten Röntgenstrahlungsquelle andere spektrale Charakteristik aufweist, insbesondere das Maximum des Spektrums bei einem anderen Energiewert hat.

Die Röntgenstrahlungsdetektorvorrichtung weist weiterhin wenigstens einen zweiten Zeilendetektor mit einer zweiten diskreten Ortsauflösung auf, die kleiner ist als die erste diskrete Ortsauflösung, wobei der wenigstens eine zweite Zeilendetektor die Röntgenstrahlung entlang einer oder mehrerer paralleler zweiter Erfassungslinien, welche parallel zu den ersten Erfassungslinien verlaufen, über die vorgegebene Erfassungsbreite diskret spektral aufgelöst detektiert und zweite Bilddaten erzeugt, wobei die Röntgenstrahlungsquanten zur spektralen Auflösung, abhängig von deren Energie, einer Vielzahl von Energiekanälen zugeordnet werden, und wobei die zweiten Bilddaten für jeden diskreten Ort einen Spektralwert für jeden Energiekanal umfassen.

Jeder der Zeilendetektoren kann auch mehrere Zeilen aufweisen. So können mehrere Bildzeilen eines zu scannenden Produkts gleichzeitig aufgenommen und ausgelesen werden. Die mehreren Zeilen können auch zur Realisierung des Time-Delay-Integration-Verfahrens (TDI-Verfahren) verwendet werden, um das Signal-Rauschverhältnis zu verbessern.

Die Bildsignale aller Zeilendetektoren sind der Auswerte- und Steuereinheit zugeführt, welche unter anderem auch zur Auswertung der ersten und zweiten Bilddaten derart ausgebildet ist, dass wenigstens ein vorgegebenes Merkmal des zu untersuchenden Produkts durch eine Kombination der in den ersten und zweiten Bilddaten enthaltenen Informationen mit der ersten diskreten Ortsauflösung detektiert wird. Die Auswerte- und Steuereinheit kann als Kombination von Hard- und Software ausgebildet sein und insbesondere eine Bildverarbeitungseinheit mit einem ausreichend schnellen Bildverarbeitungsprozessor umfassen. Die Bildverarbeitungseinheit erzeugt aus den Bilddaten der Zeilendetektoren ein oder mehrere Bilder und analysiert die Bilder hinsichtlich wenigstens eines vorgegebenen Merkmals. Dabei verwendet die Auswerte- und Steuereinheit bzw. die Bildverarbeitungseinheit Informationen, die der zweite, d. h. der spektral auflösende Zeilendetektor, liefert, um das zu analysierende Gesamtbild zu erzeugen und/oder ein erzeugtes Gesamtbild zu analysieren. Auf diese Weise können die Vorteile des spektral auflösenden Zeilendetektors zur Erzeugung eines Gesamtbildes und/oder zur Analyse eines Gesamtbildes, in welchem zumindest auch die Informationen der von dem wenigstens einen ersten, nicht spektral auflösenden Zeilendetektors gelieferten Bilddaten enthalten sind, genutzt werden.

Dieser Vorteil kann insbesondere auch dann genutzt werden, wenn die zweite diskrete Ortsauflösung des wenigstens einen spektral auflösenden Zeilendetektors deutlich geringer ist als die erste diskrete Ortsauflösung des wenigstens einen nicht spektral auflösenden Zeilendetektors. Bei dem wenigstens einen vorgegebenen Merkmal, auf welches hin die Auswerte- und Steuereinheit ein zuvor erzeugtes Gesamtbild untersucht, kann es sich um die Position eines Fremdkörpers innerhalb des Produkts, das Material des Fremdkörpers, die Dicke des Produkts, das Material des Produkts, die Dicke von Materialschichten des Produkts, oder die Position von Überlappungsbereichen von mehreren Produkten handeln.

Nach einer bevorzugten Ausführungsform der Erfindung sind die Zeilendetektoren in einem gemeinsamen Gehäuse vorgesehen. Anstelle eines Gehäuses können die Zeilendetektoren selbstverständlich auch an einem beliebigen, nicht geschlossenen Träger in einer festen Position zueinander angeordnet sein. Die Zeilendetektoren (mit oder ohne Gehäuse; mit oder ohne Träger) können auch in einem gemeinsamen Strahlenschutzgehäuse vorgesehen sein. Hierdurch kann eine Vorrichtung zur Röntgeninspektion von bewegten Produkten auf einfache Weise und auch kostengünstig, insbesondere was den Strahlenschutz anbelangt, realisiert werden. Nach einer Ausführungsform der Erfindung steuert die Auswerte- und Steuereinheit den wenigstens einen ersten und den zweiten Zeilendetektor derart an, dass eine synchrone Erfassung der Bilddaten an den ersten und zweiten Erfassungslinien abhängig von der Geschwindigkeit der Relativbewegung zwischen dem zu untersuchenden Produkt und der Röntgenstrahlungsdetektorvorrichtung erfolgt, um an den ersten und zweiten Erfassungslinien jeweils Zeilen-Bilddaten zu erhalten, die im Wesentlichen demselben Strahlengang durch das zu untersuchende Produkt entsprechen. Bei einer solchen synchronisierten Erzeugung der Bilddaten der einzelnen Zeilendetektoren kann ein Gesamtbild mit einem geringeren Aufwand erzeugt werden, da hier allenfalls eine Anpassung, z.B. Interpolation der Bilddaten (z.B. nur eines Zeilendetektors), in der Richtung der Erfassungslinien erforderlich ist. Dagegen können die Bilddaten der Zeilen der Zeilendetektoren direkt kombiniert, z.B. gewichtet addiert werden. Die Bewegungsgeschwindigkeit muss hierzu ausreichend genau bekannt sein, ebenso der Abstand der Zeilendetektoren in der Bewegungsrichtung.

Nach einer anderen Ausführungsform steuert die Auswerte- und Steuereinheit den wenigstens einen ersten und den zweiten Zeilendetektor derart an, dass eine asynchrone Datenerfassung an den ersten und zweiten Erfassungslinien erfolgt, wobei an den ersten und zweiten Erfassungslinien jeweils Zeilen-Bilddaten mit einer Zeilen-Abtastrate erfasst werden, die vorzugsweise gleich oder größer ist als die Abmessung der Pixel des jeweiligen Zeilendetektors in der Richtung der Relativbewegung dividiert durch die Geschwindigkeit der Relativbewegung. Damit kann das zu untersuchende Produkt lückenlos (in der Bewegungsrichtung gesehen) abgetastet werden. Bei der Erzeugung eines Gesamtbildes aus den Bilddaten mehrerer Zeilendetektoren muss hier erforderlichenfalls, auch in der Bewegungsrichtung gesehen, eine Anpassung der Bilddaten, beispielsweise durch Interpolation, vorgenommen werden.

Es sei an dieser Stelle angemerkt, dass die Auswerte- und Steuereinheit bzw. die Bildverarbeitungseinheit bei der Erzeugung eines Gesamtbildes aus den Bilddaten zweier oder mehrerer Zeilendetektoren jegliche erforderliche Operation anwenden kann, um die Abstände zwischen den Zeilendetektoren, deren unterschiedliche Pixel-Pitch, Winkelfehler zwischen den Zeilendetektoren, einen unterschiedlichen Abstand der Zeilendetektoren von der Röntgenstrahlungsquelle und dergleichen zu korrigieren. Als erforderliche Operationen kommen dabei insbesondere eine (lineare oder nichtlineare) Interpolation, eine geometrische Transformation (zur Korrektur von Winkelfehlern bei nicht-parallelen Erfassungslinien) und eine Gewichtung der Bilddaten (z.B. bei unterschiedlichen Detektorempfindlichkeiten) in Frage.

Bei der asynchronen Erfassung der Bilddaten des wenigstens einen ersten, nicht spektralen Zeilendetektors und des zweiten spektralen Zeilendetektors kann die Auswerte- und Steuereinheit derart ausgebildet sein, dass die Bilddaten als einziger Datenstrom zur Bildverarbeitungseinheit geführt werden. Dies bietet sich insbesondere an, wenn die Auswerte- und Steuereinheit dezentral ausgebildet ist und die Bildverarbeitungseinheit weiter von einer Datenerfassungseinheit, welcher die Bilddaten der einzelnen Zeilendetektoren als einzelne Datenströme zugeführt sind, entfernt ist. Die Datenerfassungseinheit kann die Bilddaten hierzu mit Zeitinformationen versehen, welche die Erfassungszeiten in absoluter Form oder relativ zu einem Referenzzeitpunkt beinhalten. Beispielsweise kann in dem gemeinsamen Bilddatenstrom jeder Bildzeile eine Zeitinformation zugeordnet sein. Die Bilddaten von Bildzeilen der verschiedenen Zeilendetektoren können ebenfalls im Datenstrom in geeigneter Weise als von einem bestimmten Zeilendetektor stammend gekennzeichnet sein. Eine solche Datenübertragung kann selbstverständlich auch bei einer synchronen Abtastung verwendet werden.

Wie bereits vorstehend kurz erläutert, kann die Auswerte- und Steuereinheit aus den Bilddaten des wenigstens einen ersten Zeilendetektors und des zweiten Zeilendetektors ein Gesamtbild erstellen, insbesondere durch Interpolation und/oder geometrische Transformation der Bilddaten des wenigstens eine zweiten Zeilendetektors und/oder der Bilddaten des wenigstens einen ersten Zeilendetektors. Damit beinhaltet das Gesamtbild Informationen der beiden verschiedenen Zeilendetektortypen.

Nach einer weiteren Ausführungsform der Erfindung umfassen der wenigstens eine erste Zeilendetektor und der zweite Zeilendetektor in der Richtung der betreffenden Erfassungslinie mehrere gleichartige, an Stoßstellen aneinandergereihte Module, wobei die Stoßstellen des wenigstens eine ersten Zeilendetektors und die Stoßstellen des zweiten Zeilendetektors gegeneinander versetzt sind. Damit lassen sich die Blindstellen des einen Zeilendetektors durch die Bilddaten des jeweils anderen Zeilendetektors zumindest teilweise ausgleichen. Allerdings kann der zweite Zeilendetektor in vielen Fällen nicht die höhere Ortsauflösung des ersten Zeilendetektors liefern und der erste Zeilendetektor nicht die spektrale Auflösung des zweiten Zeilendetektors. Insbesondere für die nachstehend erläuterten Ausführungsformen, bei welchen die Bilddaten des zweiten Zeilendetektors verwendet werden, um Teilbilddaten zu erzeugen, die ein frei wählbares Spektrum aufweisen, und diese Teilbilddaten mit den Bilddaten des ersten Zeilendetektors zu einem Dual-Energy Gesamtbild kombiniert werden, oder bei welchen die Bilddaten des zweiten Zeilendetektors verwendet werden, um Informationen betreffend Überlappungsbereiche oder die Produktdicke zu gewinnen, die dann zur Ermittlung von variablen Schwellenwerten zur Auswertung eines Gesamtbildes herangezogen werden, ist dieser Nachteil jedoch akzeptabel.

Diese Ausführungsform ermöglicht eine kleine Bauform, da die Module in einer geraden Linie angeordnet sind und die infolge der Kühlkörper baugrößeren Module des zweiten, spektral auflösenden Zeilendetektors nahe an den baukleineren Modulen des wenigstens einen ersten, nicht spektral auflösenden Zeilendetektors angeordnet werden können. Sollen derartige Blindstellen vermieden werden, so käme nur eine geschuppte Anordnung der Module in Frage, die jedoch eine deutlich vergrößerte Bauform zur Folge hätte.

Nach einer weiteren Variante der Erfindung kann die Auswerte- und Steuereinheit für jeden diskreten Ort die Spektralwerte der Energiekanäle der Bilddaten des zweiten Zeilendetektors gewichten, insbesondere mit jeweils einem Faktor multiplizieren, und die gewichteten Spektralwerte zu einem Summen-Spektralwert addieren. Auf diese Weise kann die Auswerte- und Steuereinheit aus den ersten Bilddaten des wenigstens einen ersten Zeilendetektors und den derart verarbeiteten zweiten Bilddaten des zweiten Zeilendetektors Dual-Energy-Bilddaten oder Multiple-Energy-Bilddaten erzeugen. Dabei kann die Auswerte- und Steuereinheit bzw. die Bildverarbeitungseinheit das Spektrum der so verarbeiteten zweiten Bilddaten durch eine beliebige Gewichtung frei wählen (eine Gewichtung mit dem Faktor Null entspricht dabei einer scharfen Begrenzung eines entsprechenden spektralen Bereichs) und so ein Dual-Energy-Bild erzeugen, welches zur Erkennung von ganz bestimmten Merkmalen des zu untersuchenden Produkts angepasst ist. Beispielsweise kann das Spektrum der verarbeiteten zweiten Bilddaten zur Erkennung von Fremdkörpern aus einem bestimmten Material angepasst werden.

Die Gewichtung kann beispielsweise so erfolgen, dass ein bestimmter spektraler Bereich, z.B. die Energiekanäle von 20 bis 40 keV, mit dem Faktor 1 gewichtet werden und alle anderen Energiekanäle durch eine Gewichtung mit Null ausgeblendet werden. Ein derart scharf begrenztes Spektrum in einem unteren Energiebereich ließe sich mit einem Filter für die Röntgenstrahlung nicht erzeugen, da diese Filter zum einen nur als Hochpassfilter verfügbar sind und zudem eine geringe Flankensteilheit aufweisen.

Damit ermöglicht die Erfindung auf diese Weise ein Dual-Energy-Verfahren, bei dem der meist höher ortsauflösende Zeilendetektor das Spektrum der Röntgenstrahlung insgesamt detektiert und der meist niedriger ortsauflösende Zeilendetektor ein beliebig und flexibel wählbares Spektrum liefert. Dieses Spektrum kann zudem nach der spektral aufgelösten Erfassung der Röntgenstrahlung beliebig gewählt werden.

Dieses Prinzip lässt sich auch dadurch realisieren, dass der spektral auflösende Zeilendetektor seinerseits eine Vorverarbeitung ausführt. Beispielswiese kann der spektral Zeilendetektor nur ausgewählte Energiekanäle in Form des Bilddatensignals auslesen, was einer Gewichtung der nicht ausgelesenen Energiekanäle mit dem Faktor Null entspricht. Die Gewichtung der ausgelesenen Energiekanäle und die Bestimmung eines Summen-Spektralwert kann dann wieder die Auswerte- und Steuereinheit entsprechend eines vorbestimmten Gewichtungsprofils vornehmen.

Der spektral auflösende Zeilendetektor kann auch so ausgebildet sein, dass nicht nur bestimmte Energiekanäle

EP 3 734 259 B1

(einzelnen) ausgelesen werden, sondern dass der Zeilendetektor gleichzeitig ein Aufaddieren der Spektralwerte der einzelnen Kanäle zu einem Summen-Spektralwert vornimmt. Der Summen-Spektralwert kann dann von der Auswerte- und Steuereinheit (mit einem einzigen Faktor) gewichtet werden. Der spektral auflösende Zeilendetektor kann hierzu auch so ausgebildet sein, dass die spektrale Breite der Energiekanäle einstellbar ist. Auf diese Weise ist es möglich (durch manuelle Eingabe oder eine Eingabe seitens der Auswerte- und Steuereinheit), den spektral auflösenden Sensor so anzusteuern, dass er einen Spektralwert für einen einstellbaren spektralen Bereich liefert, beispielsweise einem spektralen Bereich von 20 keV bis 40 keV. Dieser spektrale Wert kann dann direkt von der Auswerte- und Steuereinheit gewichtet und zur Erzeugung eines Dual-Energy-Bildes verwendet werden.

Es sei an dieser Stelle bemerkt, dass die Auswerte- und Steuereinheit durch die Verwendung eines Gewichtungsprofils, welches für jeden Energiekanal einen Gewichtungsfaktor aufweist, eine beliebig wählbare Gewichtung des von dem spektral auflösenden Sensor gelieferten Spektrums (pixelweise) vornehmen kann. Die Gewichtung kann dabei gleichzeitig eine Auswahl eines Energiekanals bewirken, wenn der jeweilige Gewichtungsfaktor ungleich Null ist, und eine tatsächliche Gewichtung des Spektralwert des betreffenden Energiekanals mit dem jeweiligen Gewichtungsfaktor. Sind die Gewichtungsfaktoren für benachbarte Energiekanäle, die zur Erzeugung von Teilbilddaten für ein Dual-Energy-Bild verwendet werden, konstant, so können die spektral Werte dieser Energiekanäle auch erst zu einem Summen-Spektralwert addiert werden, bevor der Summen Spektralwert mit dem (konstanten) Gewichtungsfaktor zur Erzeugung des Dual-Energy-Bildes verwendet wird.

Nach einer Ausführungsform der Erfindung können in der Auswerte- und Steuereinheit Informationen gespeichert sein oder die Auswerte- und Steuereinheit kann Zugriff auf Informationen haben, welche Gewichtungen von Energiekanälen für das Detektieren eines oder mehrerer bestimmter vorgegebener Merkmale des zu untersuchenden Produkts bevorzugt geeignet sind. Insbesondere können diese Informationen in Form von Gewichtungsprofilen vorliegen, wobei ein Gewichtungsprofil jedem Energiekanal einen (grundsätzlich frei wählbaren) Gewichtungsfaktor zuordnen kann. Jedem Gewichtungsprofil kann ein bestimmtes Merkmal zugeordnet werden, welches unter Verwendung des mit diesem Profil erzeugten Dual-Energy-Gesamtbildes in vorteilhafter Weise, insbesondere mit gutem Kontrast, detektierbar ist. Beispielsweise können mit einem bestimmten Gewichtungsprofil Fremdkörper aus einem bestimmten Kunststoff, z.B. aus Polyethylen, mit gutem Kontrast detektiert werden, während sich ein anderes Gewichtungsprofil für das Detektieren von Knochensplittern in dem betreffenden Gesamtbild besonders eignet.

Die Auswerte- und Steuereinheit kann die Bilddaten des wenigstens einen ersten Zeilendetektors und die Bilddaten des zweiten Zeilendetektors für ein bestimmtes zu untersuchendes Produkt auch mehrfach auswerten, insbesondere für das Detektieren verschiedener vorgegebener Merkmale, wobei für jede Auswertung jeweils Dual-Energy-Bilddaten oder Multiple-Energy-Bilddaten bzw. jeweils ein entsprechendes Gesamtbild unter Verwendung einer anderen Gewichtung bzw. eines anderen Gewichtungsprofils für die Spektralwerte der Energiekanäle der Bilddaten des zweiten Zeilendetektors erzeugt werden. Damit können durch einen einzigen Scan des Produkts verschiedenartige Auswertungen vorgenommen werden. Beispielsweise kann ein Gesamtbild, das unter Verwendung eines ersten Gewichtungsprofils erzeugt wurde, auf das Vorhandensein von Fremdkörpern aus Metall untersucht werden, und ein Gesamtbild, das unter Verwendung eines zweiten Gewichtungsprofils erzeugt wurde, auf das Vorhandensein von Fremdkörpern aus Kunststoff.

Nach einer weiteren Ausführungsform kann die Auswerte- und Steuereinheit die Bilddaten des zweiten Zeilendetektors zur Erkennung von Produktbereichen mit unterschiedlichen Schichtdicken und/oder Bereichen von überlappenden Produkten auswerten und derartige Bereiche in den Bilddaten des wenigstens einen ersten Zeilendetektors oder in kombinierten Bilddaten identifizieren. Zur Inspektion der so identifizierten Bereiche kann die Auswerte- und Steuereinheit einen Schwellenwert verwenden, der abhängig von den Grauwerten eines oder mehrerer dieser Bereiche in dem betreffenden Gesamtbild gesetzt wird. Beispielsweise kann der mittlere Grauwert eines solchen örtlichen Bereichs in dem Gesamtbild ermittelt werden und der Schwellenwert, ab dem die Auswerte- und Steuereinheit einen Fremdkörper erkennt, abhängig von diesem Grauwert nach einer vorgegeben Vorschrift bestimmt werden. Ein solcher dynamischer Schwellenwert bietet den Vorteil einer unabhängig von der Dicke des Produkts oder unabhängig von überlappenden Produktbereichen zutreffenden Erkennung von Fremdkörpern oder anderen Merkmalen eines zu untersuchenden Produkts.

Durch eine geeignete Auswertung der Bilddaten des zweiten, spektral auflösenden Zeilendetektors kann die Dicke eines bekannten Materials festgestellt werden. Weiterhin kann durch eine geeignete Auswertung dieser Daten sogar festgestellt werden, um welches Material bzw. um welche Materialkombination es sich handelt. Denn jedes Material erzeugt bei der Durchstrahlung mit einem Röntgenspektrum, welches eine ausreichende spektrale Breite von z.B. 20 bis 160 keV aufweist, einen charakteristischen Verlauf des Energiespektrums. So kann pixelweise durch einen Vergleich des Energiespektrums, das ohne ein Produkt detektiert wurde, und des Energiespektrums, das mit Produkt detektiert wurde, der für das betreffende (zunächst unbekannte) Material charakteristische Verlauf der Dämpfung der Röntgenstrahlung als Funktion der Energie bestimmt werden. Durch einen Vergleich dieses charakteristischen Verlaufs mit bekannten charakteristischen Verläufen für jeweilige Materialien kann das Material bestimmt werden. Die Auswerte- und Steuereinheit kann hierzu jedes detektierte Energiespektrum mit gespeicherten charakteristischen Verläufen von Produkten vergleichen, beispielsweise durch eine Korrelationsanalyse.

Während der Verlauf eines detektierten Energiespektrums auf das Material schließen lässt, können aus der Dämpfung der detektierten Strahlung (in einem Energiekanal oder mehreren oder allen integrierten Energiekanälen) in Kenntnis des jeweiligen Materials die Dicke des Produkts und auch Dickenunterschiede in Abhängigkeit vom Ort bestimmt werden. Auf diese Weise können auch Überlappungsbereiche von zwei oder mehreren Produkten bestimmt werden. Hierzu kann die Auswerte- und Steuereinheit auf Informationen zugreifen, die den Zusammenhang zwischen der Dämpfung und der Dicke des betreffenden Materials beinhalten. Hierbei kann es sich um Werte für den energie-abhängigen Absorptionskoeffizienten $\mu(E)$ handeln, wobei mit E die Energie der Röntgenstrahlung bezeichnet ist. Damit kann unter Verwendung des Lambert-Beer'schen Gesetzes die Dicke eines bekannten Materials ermittelt werden.

Nach einer weiteren Ausführungsform der Erfindung ermittelt die Auswerte- und Steuereinheit zur Überwachung des Zustands eines ausgewählten Zeilendetektors Bilddaten des zu überwachenden Zeilendetektors und Bilddaten eines als Referenz-Zeilendetektors ausgewählten Zeilendetektors, vorzugsweise jeweils ohne Vorhandensein eines Produkts, wobei die Auswerte- und Steuereinheit zur Ermittlung der Bilddaten des zweiten Zeilendetektors die Spektralwerte eines, mehrerer oder aller Energiekanäle aufsummiert, und wobei die Auswerte- und Steuereinheit die Bilddaten des zu überwachenden Zeilendetektors mit Bilddaten des Referenz-Zeilendetektors vergleicht und bei Feststellen unzulässiger Abweichungen der Bilddaten des zu überwachenden Zeilendetektors von den Bilddaten des Referenz-Zeilendetektors ein "Nicht-Bereit-Signal" erzeugt.

Die Auswahl der Energiekanäle, deren Spektralwerte zu einem Summen-Spektralwert addiert werden, kann dabei so getroffen werden, dass sich für beide Zeilendetektoren derselbe Verlauf der spektralen Empfindlichkeit ergibt. Ist beispielsweise bekannt, dass ein zu überwachender, nicht spektral auflösende Zeilendetektor eine Empfindlichkeit im Bereich von 20 keV bis 80 keV aufweist und der spektral auflösende Zeilendetektor (d. h. der Referenz-Zeilendetektor) eine Empfindlichkeit von 20 keV bis 160 keV, so wird man nur diejenigen Energiekanäle des spektral auflösenden Zeilendetektors für die Ermittlung des betreffenden Summen-Spektralwert verwenden, die den Empfindlichkeitsbereich des nicht spektral auflösenden Zeilendetektors abdecken, also die Energiekanäle im Bereich von 20 keV bis 80 keV. Diese Vorgehensweise ist selbstverständlich unabhängig davon, ob der spektral auflösende Zeilendetektor als Referenz-Zeilendetektor dient oder den zu überwachenden Zeilendetektor darstellt.

Anstelle einer Addition der Spektralwerte der betreffenden Energiekanäle durch die Auswerte- und Steuereinheit, kann der spektral auflösende Sensor selbst diese Funktionalität bereitstellen. Insbesondere kann er nur die ausgewählten Energiekanäle im Bilddatensignal bereitstellen oder bereits den Summen-Spektralwert im Bilddatensignal an die Auswerte- und Steuereinheit übermitteln.

Es ist darüber hinaus möglich, einen bekannten spektralen Empfindlichkeitsverlauf eines nicht spektral auflösenden Zeilendetektors (innerhalb der spektralen Breite der zu agierenden Energiekanäle des spektral auflösenden Zeilendetektors) zu berücksichtigen. Hierzu kann die Auswerte- und Steuereinheit die zu agierenden Spektralwerte der jeweiligen Energiekanäle vor der Addition in geeigneter Weise gewichten. Auf diese Weise kann die spektrale Empfindlichkeit des spektral auflösenden Zeilendetektors der spektralen Empfindlichkeit eines nicht spektral auflösenden Zeilendetektors angenähert werden.

Unter Anwendung dieses Überwachungsverfahrens kann insbesondere die Bereitschaft des zweiten, spektral auflösenden Zeilendetektors überprüft und festgestellt werden, wenn dieser eingeschaltet wird. Denn, wie vorstehend erläutert, kann es längere Zeit, z.B. 30 min dauern, bis der spektral auflösende Sensor ein ausreichende Ladungstrennung erreicht hat. Hierzu kann pixelweise der Grauwert eines nicht spektral auflösenden Sensors mit dem entsprechenden Grauwert des spektral auflösenden Sensors verglichen werden, wobei sich der Grauwert des spektral auflösenden Sensors durch Aufsummieren der Spektralwerte aller Energiekanäle (für jedes Pixel) ergibt. Kennt die Auswerte- und Steuereinheit das Verhältnis der einander entsprechenden Pixel-Grauwerte der beiden Sensoren bei voller (korrekter) Funktionsfähigkeit, so kann diese das "Nicht-Bereit-Signal" erzeugen, wenn der Grauwert eines Vergleichspixels des nicht spektral auflösenden Zeilendetektor um mehr als einen zulässigen Wert von dem Grauwert des entsprechenden Vergleichspixels des spektral auflösenden Zeilendetektors aufweist.

Das "Nicht-Bereit-Signal" kann bereits dann erzeugt werden, wenn für ein einziges Paar von einander entsprechenden Vergleichspixeln eine unzulässige Abweichung festgestellt wird. Es sei darauf hingewiesen, dass ein "Nicht-Bereit-Signal" in allen Fällen erzeugt werden kann, in denen die Bilddaten des zu überwachenden Zeilendetektors um mehr als einen vorgegebenen Wert von den Bilddaten des Referenz-Zeilendetektors abweichen.

Die Auswerte- und Steuereinheit die Referenzbilddaten kann diesen pixelweisen Vergleich, unter Verwendung von einander entsprechenden Vergleichspixeln durchführen. Ein Vergleichspixel kann dabei aus einem oder mehreren ganz oder anteilig zu berücksichtigenden Pixeln eines Zeilendetektors bestehen. Die Vergleichspixel werden so gewählt, dass zwei einander entsprechende Vergleichspixel dieselbe Strecke auf den jeweiligen Erfassungslinien abdecken, d.h. die Breite jedes Paars von einander entsprechenden Vergleichspixeln ist identisch, wobei die Strecke, welche von einem Vergleichspixel auf der betreffenden Erfassungslinie abgedeckt ist durch eine Parallelverschiebung der Strecke, welche das jeweils entsprechende Vergleichspixel auf der betreffenden anderen Erfassungslinie abdeckt, hervorgeht. Zur Bestimmung des Grauwertes eines Vergleichspixels werden die Werte aller Pixel, die ganz oder teilweise innerhalb der Breite des Vergleichspixels liegen, aufaddiert, wobei der Grauwert eines Pixels, welches nicht mit der gesamten Breite innerhalb der Breite des jeweiligen Vergleichspixels liegt, mit dem Verhältnis gewichtet wird, mit welchem das betreffende Pixel mit seiner Breite innerhalb der Breite des Vergleichspixels liegt.

Werden beispielsweise die breiteren Pixel des niedriger ortsauflösenden zweiten Zeilendetektors jeweils als Vergleichspixel verwendet, und liegen jeweils ein schmäleres Pixel des höher ortsauflösenden ersten Zeilendetektors ganz und zwei Pixel des höher ortsauflösenden ersten Zeilendetektors mit ihrer halben breite innerhalb der Breite des Vergleichspixels des zweiten Zeilendetektors, so wird der Grauwert des entsprechenden Vergleichspixel des ersten Zeilendetektors, welches aus dem ganzen und den zwei halben Pixeln der ersten Zeilendetektors bestehen, durch die Addition des Grauwertes des ganzen Pixels und der mit einem Faktor von 0,5 gewichteten Grauwerte der jeweils halben Pixel bestimmt.

Auf diese Weise kann ein einfacher Vergleich der Bilddatensignale des als Referenz-Zeilendetektors bestimmten Zeilendetektors und des zu überwachenden Zeilendetektors erfolgen.

Die erfindungsgemäße Vorrichtung zur Röntgeninspektion von Produkten, insbesondere von Lebensmitteln, ist dazu ausgebildet, ein in einer Bewegungsrichtung mit einer vorgegebenen Bewegungsgeschwindigkeit bewegtes zu untersuchendes Produkt mit der erzeugten Röntgenstrahlung zu durchstrahlen.

Nach einer Ausführungsform sind der wenigstens eine erste Zeilendetektor und der zweite Zeilendetektor derart angeordnet, dass die eine oder mehreren zweiten Erfassungslinien des zweiten Zeilendetektors nicht im Schatten des wenigstens einen ersten Zeilendetektors liegen und die eine oder mehreren ersten Erfassungslinien des ersten Zeilendetektors nicht im Schatten des zweiten Zeilendetektors liegen. Dabei können die eine oder mehreren ersten und zweiten Erfassungslinien einen möglichst geringen Abstand in der Bewegungsrichtung des zu untersuchenden Produkts aufweisen. Hierdurch ergibt sich eine Abtastung von Produktbereichen durch die wenigstens zwei Zeilendetektoren, die, in Bewegungsrichtung gesehen, einen möglichst geringen Abstand aufweisen. Schwankungen in der Bewegungsgeschwindigkeit, die der Auswerte- und Steuereinheit nicht bekannt sind, haben daher einen geringen Einfluss auf die Erzeugung eines Gesamtbildes, da der identische Produktbereich in einem nur sehr kurzen zeitlichen Abstand durchstrahlt und abgetastet wird. Zudem verläuft der Strahlengang der auf annähernd demselben Weg durch das Produkt und es ergibt sich eine geringe Abmessung der Detektorvorrichtung.

Dabei können der wenigstens eine erste Zeilendetektor und der zweite Zeilendetektor, in Strahlungsrichtung gesehen, überlappend angeordnet sein, wobei die eine oder mehreren ersten und zweiten Erfassungslinien einen unterschiedlichen Abstand von der wenigstens einen Röntgenstrahlungsquelle aufweisen. Dieser Abstand kann, abhängig von der Baugröße des wenigstens einen ersten und des zweiten Zeilendetektors, möglichst gering gewählt sein. Damit wird eine sehr geringe Bauhöhe erreicht.

Die Erfindung wird nachstehend unter Bezugnahme auf die Zeichnung näher erläutert. In der Zeichnung zeigen:

Fig. 1 eine schematische Darstellung einer ersten Ausführungsform einer Vorrichtung zur Inspektion von Produkten mit einer ersten Ausführungsform einer Röntgenstrahlungsdetektorvorrichtung mit zwei nicht spektral auflösenden Zeilendetektoren und einem spektral auflösenden Zeilendetektor;

Fig. 2 eine Seitenansicht (Fig. 2a) einer und eine Draufsicht (Fig. 2b) auf eine weitere Ausführungsform einer Röntgenstrahlungsdetektorvorrichtung, welche eine Kombination aus einem nicht spektral auflösenden und einem spektral auflösenden Zeilendetektor umfasst;

Fig. 3 einen Ausschnitt einer schematischen Draufsicht auf die beiden Zeilen einer weiteren Ausführungsform einer Röntgenstrahlungsdetektorvorrichtung ähnlich der Ausführungsform in Fig. 2; und

Fig. 4 einen schematischen Ausschnitt einer Variante der Ausführungsform in Fig. 3.

Fig. 5 ein beispielhaftes Energiespektrum, das von einem Pixel eines spektral auflösenden Zeilendetektor erzeugt wurde;

Fig. 6 ein Gewichtungsprofil zur Gewichtung der Energiekanäle eines Energiespektrums gem. Fig. 5;

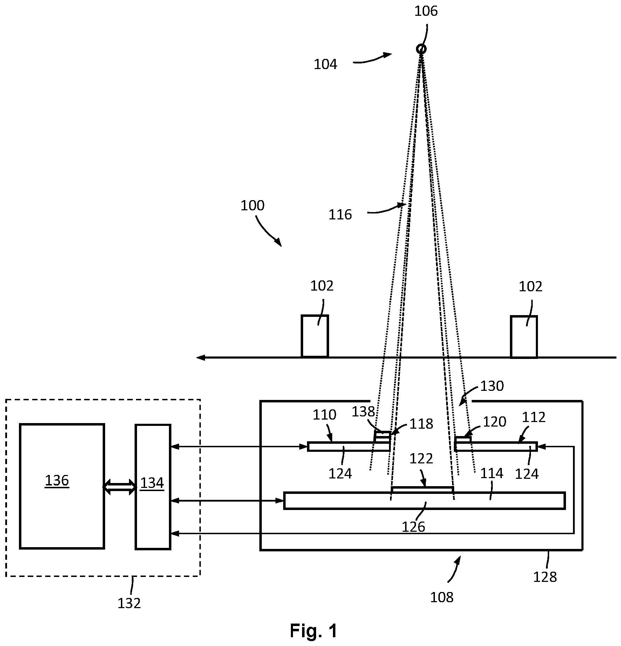

Fig. 1 zeigt eine schematische Darstellung einer ersten Ausführungsform einer Vorrichtung 100 zur Röntgeninspektion von Produkten 102, insbesondere von Lebensmitteln, mit einer Strahlungserzeugungsvorrichtung 104 mit wenigstens einer Röntgenstrahlungsquelle 106 und mit einer Röntgenstrahlungsdetektorvorrichtung 108 mit zwei nicht spektral auflösenden Zeilendetektoren 110, 112 und einem spektral auflösenden Zeilendetektor 114.

Die Röntgenstrahlungsquelle 106 erzeugt einen fächerförmigen Röntgenstrahl 116, welcher eine Mittelebene E aufweist, die senkrecht zu einer Bewegungsrichtung B steht, in welcher die zu untersuchenden Produkte 102 durch den Röntgenstrahl 116 bewegt werden. Der Röntgenstrahl 116 weist in der Bewegungsebene E einen Öffnungswinkel auf, der so ausgebildet ist, dass das zu untersuchende Produkt 102 in seiner gesamten Breite von dem Röntgenstrahl 116 durchstrahlt wird. Zur Bewegung des Produkts 102 kann eine Fördervorrichtung (nicht dargestellt), beispielsweise ein Transportband, vorgesehen sein.

Die nicht spektral auflösenden Zeilendetektoren 110, 112 weisen jeweils eine einzige Zeile 118, 120 von Pixeln auf, die die Röntgenstrahlung des Röntgenstrahls 116 entlang jeweils einer Erfassungslinie detektiert. In diesem Zusammenhang wird von einer Erfassungslinie gesprochen, auch wenn die Pixel der jeweiligen Zeile 118, 120 eine endliche Ausdehnung aufweisen. Die nicht spektral auflösenden Zeilendetektoren 110, 112 weisen eine höhere diskrete Ortsauflösung auf, als der spektral auflösende Zeilendetektor 114, der ebenfalls eine Zeile 122 von Pixeln umfasst, die die Röntgenstrahlung des Röntgenstrahls 116 entlang einer Erfassungslinie detektiert. Die diskrete Ortsauflösung der Zeilendetektoren 110, 112, d. h. die Anzahl der Pixel pro Längeneinheit bzw. die Pixel-Pitch, kann beispielsweise 0,2 mm betragen und damit viermal so groß sein wie die Pixel-Pitch des Zeilendetektors 114, die beispielsweise 0,8 mm betragen kann.

Die nicht spektral auflösenden Zeilendetektoren 110, 112 sind mit ihren Detektorzeilen 118, 120, welche jeweils an einem Rand eines Trägers 124 vorgesehen sind, einander zugewandt, wobei, in Bewegungsrichtung B gesehen, ein Abstand zwischen den Detektorzeilen 118, 120 verbleibt. Dieser Abstand entspricht in etwa der Breite der Detektorzeile 122 des spektral auflösenden Zeilendetektors 114. Die Detektorzeile 122 ist dabei in etwa mittig auf einem Träger 126 vorgesehen, der auch Kühlkörper und andere Komponenten tragen kann. Die Kühlkörper können auch den Träger 126 bilden. Die Detektorzeilen 118, 120 können, wie in Fig. 1 dargestellt, denselben Abstand zur Röntgenstrahlungsquelle 106 aufweisen. Der spektral auflösende Zeilendetektor 114, der infolge der erforderlichen Kühlkörper eine größere Bauform aufweist als die nicht spektral auflösenden Zeilendetektoren 110, 112, befindet sich, in der Richtung des Röntgenstrahls 116 gesehen, unterhalb der Zeilendetektoren 110, 112. Da die Röntgenstrahlung, die von den Zeilendetektoren 110, 112 und 114 detektiert wird, das Produkt 102 möglichst auf demselben Strahlenweg durchstrahlen soll, ist es vorteilhaft, die Detektorzeilen 118, 120 und 122 mit einem möglichst geringen Abstand, in der Bewegungsrichtung B gesehen, bzw. innerhalb eines möglichst kleinen Öffnungswinkels des Röntgenstrahls 116 in einer Ebene, die nicht senkrecht zur Mittelebene des fächerförmigen Röntgenstrahls 116 steht, anzuordnen.

Die Zeilendetektoren 110, 112, 114 können, wie in Fig. 1 gezeigt, in einem gemeinsamen Gehäuse 128 vorgesehen sein, welches als Strahlenschutzgehäuse ausgebildet sein kann. Das Gehäuse 128 weist an seiner Oberseite, d. h. der der Röntgenstrahlungsquelle 106 zugewandten Seite, eine Öffnung 130 auf, welche das Eindringen des Röntgenstrahls 116 in das Gehäuse in Richtung auf die Pixelzeilen 118, 120, 122 der Zeilendetektoren 110, 112, 114 ermöglicht.

Anstelle eines einzigen spektral auflösenden Zeilendetektors 114 können auch zwei oder mehrere spektral auflösende Zeilendetektoren vorgesehen sein. Dies kann dann von Vorteil sein, wenn die nicht spektral auflösenden Zeilendetektoren jeweils zur Erfassung einer unterschiedlichen maximalen spektralen Breite ausgebildet sind. Beispielsweise kann einer der spektral auflösenden Zeilendetektoren eine spektrale Breite von maximal 20 keV bis 160 keV mit einer spektralen Auflösung von 256 Energiekanälen aufweisen und ein weiterer spektral auflösende Zeilendetektor eine spektrale Breite von maximal 20 keV bis 80 keV, ebenfalls bei einer Auflösung von 256 Energiekanälen. Der weitere spektral auflösender Zeilendetektor weist somit eine doppelt so hohe spektrale Auflösung auf wie der erste spektral auflösende Zeilendetektor.

Die Zeilendetektoren 110, 112, 114 erzeugen jeweils ein Bilddatensignal, welches einer Auswerte- und Steuereinheit 132 zugeführt ist. Die Auswerte- und Steuereinheit 132 kann eine Datenerfassungseinheit 134 und eine Bildverarbeitungseinheit 136 aufweisen. Der Datenerfassungseinheit 134 sind die Bilddatensignale der Zeilendetektoren 110, 112, 114 zugeführt. Die Bilddatensignale können von der Datenerfassungseinheit 34 zu einem einzigen Signal bzw. Bilddatenstrom zusammengefasst werden, der der Bildverarbeitungseinheit 136 zugeführt ist. Die Bildverarbeitungseinheit 136 ist zur weiteren Verarbeitung und Analyse der Bilddaten ausgebildet. Die Datenerfassungseinheit 134 kann auch so ausgebildet sein, dass diese die Zeilendetektoren 110, 112, 114 in geeigneter Weise ansteuert, insbesondere hinsichtlich der Abtastzeitpunkte. Hierzu kann die Datenerfassungseinheit 134 jedem der Zeilendetektoren ein Taktsignal zuführen, wobei die Bilddatenerfassung durch die Zeilendetektoren dann synchronisiert mit dem Taktsignal erfolgen kann.

Im Rahmen einer synchronen Bilddatenerfassung kann insbesondere erreicht werden, dass die nicht spektral auflösenden Zeilendetektoren 110, 112, abhängig von der Bewegungsgeschwindigkeit des zu untersuchenden Produkts 102, Röntgenstrahlung detektieren, die das zu untersuchende Produkt, wenn auch zeitlich versetzt, im Wesentlichen auf demselben Weg durchdrungen hat. Durch eine Anordnung der Zeilendetektoren 110, 112 derart, dass die Detektorzeilen 118, 120 einen möglichst geringen Abstand aufweisen, wirken sich der Steuer- und Auswerteeinheit 132 nicht bekannte Geschwindigkeitsänderungen nicht nachteilig auf die Synchronität der Datenerfassung aus. Gleiches gilt natürlich auch für eine synchronisierte Abtastung eines Produkts 102 mittels des spektral auflösenden Zeilendetektors 114.

Die Bildverarbeitungseinheit 136 kann die von den Zeilendetektoren 110, 112, 114 erfassten Bilddaten in der nachstehenden Art und Weise verarbeiten. Bei der in Fig. 1 dargestellten Ausführungsform können die Bilddaten der nicht spektral auflösenden Zeilendetektoren 110, 112 entsprechend einem an sich bekannten Dual-Energy-Verfahren ausgewertet werden. Hierzu kann der Zeilendetektor 110 über ein Röntgenstrahlungsfilter 138 verfügen, welches im Verlauf des Strahlengangs zwischen der Röntgenstrahlungsquelle 106 und der Detektorzeile 118 vorgesehen ist. Hierdurch können mittels der nicht spektral auflösenden Zeilendetektoren 110, 112 jeweils Bilddaten erfasst werden, die jeweils einen Grauwert aufweisen, der sich durch Integration des jeweils gesamten detektierten Röntgenstrahlungsspektrums, abhängig von der Intensität der detektierten Röntgenstrahlung, ergibt. Wie an sich bekannt, kann bei einer derartigen Ausführungsform ein höherer Kontrast beim Detektieren von Fremdkörpern in einem Produkt 102 erreicht werden, wenn die Fremdkörper aus einem Material bestehen, welches einen anderen Absorptionskoeffizienten aufweist als das Produkt selbst.

Die Bilddaten des spektral auflösenden Zeilendetektors 114 liefern jedoch zusätzliche spektrale Informationen, mit denen das höher auflösende Bild, das aus den Bilddaten der nicht spektral auflösenden Zeilendetektoren 110, 112 gewonnen werden kann, verbessert werden kann bzw. mit denen aus den Bilddaten der nicht spektral auflösenden Zeilendetektoren 110, 112 ein oder mehrere höher auflösende Bilder erzeugt werden können, aus denen bestimmte Eigenschaften des zu untersuchenden Produkts, beispielsweise möglicherweise vorhandene Fremdkörper aus unterschiedlichen Materialien, besser erkennbar sind. Insbesondere können die Auswerte- und Steuereinheit 132 bzw. die Bildverarbeitungseinheit 136 bei einer Vorrichtung zur Röntgeninspektion von Produkten gemäß Fig. 1 aus den spektralen Bilddaten des spektral auflösenden Zeilendetektors 114 Informationen über das Material oder die Materialzusammensetzung des durchstrahlten Produkts gewinnen. Auch Informationen betreffend die Dicke des Materials bzw. die gesamte Dicke von mehreren Schichten des selben Materials lassen sich so gewinnen. Wie eine derartige Auswertung der Bilddaten vorgenommen werden kann, wird weiter unten erläutert.

Das Vorsehen eines spektral auflösenden Zeilendetektors hat jedoch nicht nur Vorteile in Verbindung mit zwei oder mehreren nicht spektral auflösenden Sensoren, wie dies vorstehend unter Bezugnahme auf die Ausführungsform einer Vorrichtung zur Röntgeninspektion gemäß Fig. 1 erläutert wurde. Vielmehr bietet die geeignete Auswertung der von einem spektral auflösenden Zeilendetektor gelieferten Bilddaten auch dann Vorteile, wenn diese mit Bilddaten eines einzigen nicht spektral auflösenden Zeilendetektors kombiniert werden oder wenn die Bilddaten des einzigen nicht spektral auflösenden Zeilendetektors unter Verwendung der aus den Bilddaten des spektral auflösenden Zeilendetektors gewonnenen Informationen zum Zweck einer einfachen Analyse aufbereitet werden.

Solche Ausführungsformen mit einem einzigen nicht spektral auflösenden Zeilendetektor können einen ähnlichen Aufbau aufweisen, wie in Fig. 1 dargestellt, wobei lediglich einer der beiden Zeilendetektoren 110 bzw. 112 entfällt.

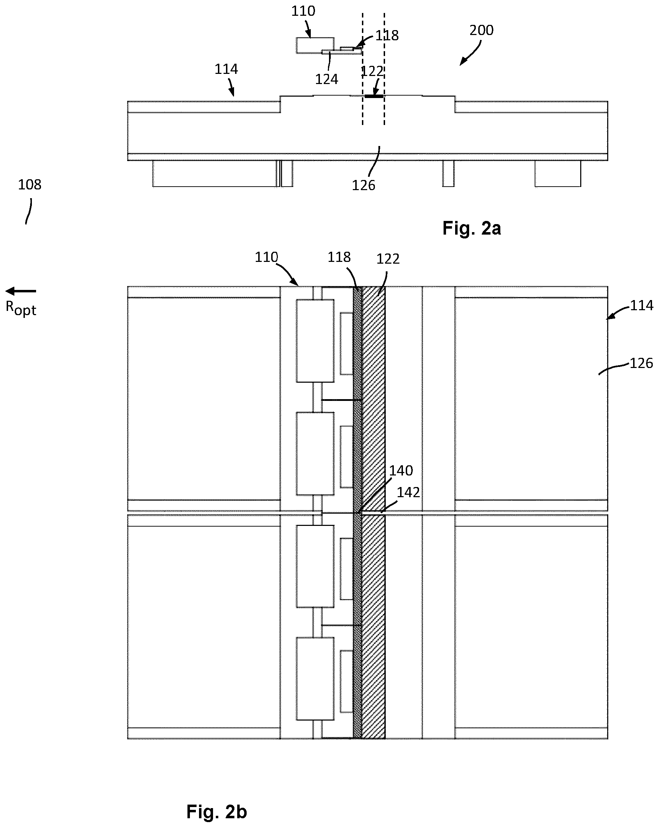

Fig. 2 zeigt schematisch die Anordnung eines nicht spektral auflösenden Zeilendetektors 110 zusammen mit einem spektral auflösenden Zeilendetektor 114 im Rahmen einer solchen Röntgenstrahlungsdetektorvorrichtung 200. Auf die Darstellung der übrigen Komponenten, insbesondere des Gehäuses sowie der Auswerte- und Steuereinheit, wurde verzichtet. Die Anordnung der Zeilendetektoren 110 und 114 erfolgt, wie auch bei der Ausführungsform in Fig. 1, so, dass die Detektorzeilen 118 und 122 einen geringen Abstand in horizontaler Richtung und auch einen geringen Abstand in vertikaler Richtung aufweisen (siehe die Seitenansicht gemäß Fig. 2a).

Aus Fig. 2b, welche die beiden Zeilendetektoren 110 und 114 in einer ausschnittsweisen Draufsicht darstellt, ist ersichtlich, dass jeder der Zeilendetektoren 110, 114 in Form von Modulen aufgebaut ist, die jeweils in Richtung der betreffenden Detektorzeile 118, 122 gekoppelt sind. Der spektral auflösende Zeilendetektor 114 besteht aus Modulen $114_i$ (der Zähler i bezeichnet eine beliebige natürliche Zahl größer gleich 1), die eine größere Breite aufweisen als die Module $110_k$ (der Zähler k bezeichnet eine beliebige natürliche Zahl größer gleich 1) des spektral auflösenden Zeilendetektors 110. Von den Modulen des spektral auflösenden Zeilendetektors 114 sind lediglich zwei Module, nämlich die Module $114_1$ und $114_2$ dargestellt. Diese können beispielsweise eine Breite (in Richtung der Detektorzeile 122 gesehen) von 10 cm aufweisen. Von den Modulen des nicht spektral auflösenden Zeilendetektors 110 sind Module $110_1$, $110_2$, $110_3$ und $110_4$ gezeigt. Diese Module weisen die halbe Breite (in Richtung der Detektorzeile 118 gesehen) auf, wie die Module des Zeilendetektors 114. Dies führt jedoch zu dem Nachteil, dass bei jeder zweiten Stoßstelle 140 der Module $110_1$, $110_2$, $110_3$ und $110_4$ des Zeilendetektors 110 auch eine Stoßstelle 142 der Module $114_1$ und $114_2$ des Zeilendetektors 114 vorliegt. Da jeder Detektor in der unmittelbaren Nachbarschaft seines Seitenrandes einen Randbereich aufweist, der nicht von Pixeln abgedeckt ist, ist die Ortsauflösung des jeweiligen Zeilendetektors an einer Stoßstelle nicht mehr gegeben. Typischerweise beträgt der Randbereich jedes Moduls, in dem keine Pixel mehr vorgesehen sind, eine Breite von ca. ein bis drei Pixeln. Damit fehlt bei jedem Zeilendetektor 110, 114 an einer Stoßstelle in den jeweiligen Bilddaten die Information über eine Breite von zwei bis sechs Pixeln.

Es bietet sich daher an, die Module der Zeilendetektoren 110, 114 so gegeneinander zu versetzen, dass die Stoßstellen der Module eines der Zeilendetektoren nicht an den Stoßstellen der Module des jeweils anderen der Zeilendetektoren vorliegen.

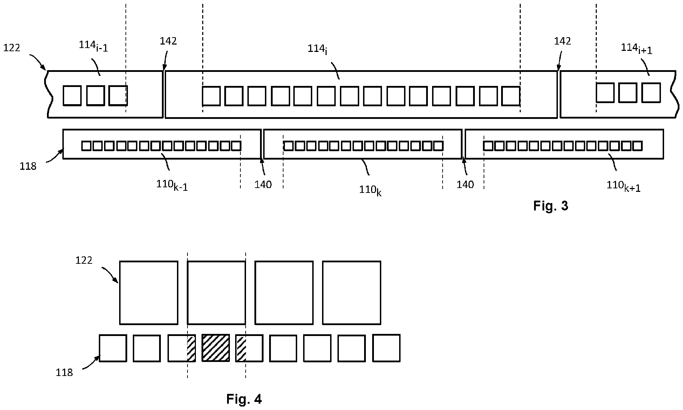

Diese Maßnahme ist nochmals deutlicher aus Fig. 3 ersichtlich, welche einen Ausschnitt der Detektorzeile 118 des spektral auflösenden Zeilendetektors 114 und der Detektorzeile 122 des nicht spektral auflösenden Zeilendetektors 114 schematisch in einer Draufsicht darstellt. Von jedem Zeilendetektor 110, 114 sind jeweils drei Module (teilweise) dargestellt. Da bei der gezeigten Ausführungsform ebenfalls die Module des nicht spektral auflösenden Zeilendetektors 110 halb so breit sind wie die Module des spektral auflösenden Zeilendetektors 114, ergibt sich durch einen Versatz der Module zueinander niemals eine Position, an welcher gleichzeitig jeweils eine Stoßstelle beider Zeilendetektoren vorliegt. Bei den Ausführungsformen der Zeilendetektoren gemäß Fig. 3 die Pixel-Pitch des spektral auflösenden Zeilendetektors 114 doppelt so groß wie die Pixel-Pitch des nicht spektral auflösenden Zeilendetektors 110. Die Kantenabmessungen der (quadratischen) Pixel beider Sensoren unterscheiden sich somit ebenfalls annähernd um den Faktor zwei.

Um zu vermeiden, dass die Blindstellen einer Detektorzeile zwischen zwei benachbarten Pixeln zusammenfallen, können die Zeilendetektoren bzw. die betreffenden Module auch derart versetzt angeordnet werden, dass insbesondere an der Position einer Blindstelle zwischen zwei benachbarten größeren Pixeln des nicht spektral auflösenden Zeilendetektors 114 nicht gleichzeitig eine Blindstelle zwischen benachbarten Pixeln des nicht spektral auflösenden Zeilendetektors 110 vorliegt. Diese Vorgehensweise ist in Fig. 4, welche einen Ausschnitt der Detektorzeilen 118, 122 anhand der schematischen, vergrößerten Darstellung in Fig. 3 zeigt, nochmals deutlicher dargestellt.

Wie vorstehend erläutert, kann die Auswerte- und Steuereinheit 132 die Bilddatensignale der Zeilendetektoren dazu verwenden, eine Überwachung eines der Zeilendetektoren (ausgewählter Zeilendetektor) durch einen Vergleich mit einem anderen der Zeilendetektoren (Referenz-Zeilendetektor) auszuführen. Dies bietet sich insbesondere zur Überwachung der Bereitschaft des spektral auflösenden Zeilendetektors 114 an, da derartige Zeilendetektoren eine längere Anlaufzeit (beispielsweise 30 Minuten) benötigen, bis brauchbare Bilddaten ausgegeben werden. Die Anlaufzeit kann insbesondere durch das erforderliche Schaffen einer ausreichenden Ladungstrennung bedingt sein.

Für einen solchen Vergleich können die Bilddaten des Referenz-Zeilendetektors, insbesondere pixelweise, mit den Bilddaten des ausgewählten Zeilendetektors verglichen werden. Der Vergleich kann beispielsweise derart erfolgen, dass die Auswerte- und Steuereinheit 136 ein Soll-Verhältnis der von einander entsprechenden Vergleichspixeln gelieferten Signalwerte kennt (beispielsweise entsprechende Soll-Verhältnisse für ein oder mehrere Vergleichspixel-Paare in der Auswerte- und Steuereinheit gespeichert sind) und ein "Nicht-Bereit-Signal" erzeugt, wenn ein oder mehrere Vergleichspixel-Paare das soll-Verhältnis nicht erfüllen.

Der Begriff "Vergleichspixel" wird in diesem Zusammenhang eingeführt, da der ausgewählte Zeilendetektor und der Referenz-Zeilendetektor gegebenenfalls unterschiedliche Pixelgrößen bzw. eine unterschiedlich Pixel-Pitch aufweisen (siehe die Fig. 3 und 4). Als Vergleichspixel können insbesondere die Pixel desjenigen dieser beiden Zeilendetektoren verwendet werden, welche die größeren geometrischen Abmessungen aufweisen. Als Wert des Vergleichspixels dieses Zeilendetektors können dann unmittelbar (ohne weitere Verarbeitung) die jeweiligen Pixelwerte verwendet werden. Weist der jeweils andere Zeilendetektor eine andere Pixelgröße auf, so kann die Auswerte- und Steuereinheit 136 sämtliche (kleineren) Pixel dieses Zeilendetektors zur Ermittlung eines entsprechenden Wertes für dieses Vergleichspixel heranziehen, die - in der Richtung der Detektorzeile gesehen - innerhalb der Breite des Vergleichspixels des Zeilendetektors mit den größeren Pixelabmessungen liegen.

Dies sei nochmals anhand Fig. 4 für die Überwachung des spektral auflösenden Zeilendetektors 114 mit den größeren Pixelabmessungen unter Verwendung des nicht spektral auflösenden Zeilendetektors 110 als Referenz-Detektor für eine Ausführungsform gemäß Fig. 2 näher erläutert. Als Vergleichspixel für den spektral auflösenden Zeilendetektor 114 wird jeweils ein Pixel mit seinen tatsächlichen Abmessungen, beispielsweise 0,8 mm Breite, verwendet. Diese Breite deckt die schraffierten Bereiche von drei Pixeln des nicht spektral auflösenden Zeilendetektors 110 ab, die zusammen das Vergleichspixel des Zeilendetektors 110 bilden. Zur Bildung des Soll-Verhältnisses bildet die Auswerte- und Steuereinheit 136 das Verhältnis der Pixelwerte, welches der Zeilendetektor 114 für das betreffende Pixel (bzw. Vergleichspixel) liefert, und der anteiligen Pixelwerte, welche der Zeilendetektor 110 für die drei tatsächlichen Pixel liefert, die das entsprechende Vergleichspixel des Zeilendetektors 110 bilden. Dabei wird das voll abgedeckte tatsächliche Pixel (ganz schraffiert dargestellt) mit seinem unveränderten Pixelwert berücksichtigt und die beiden äußeren, nur teilweise vom Vergleichspixel des Zeilendetektors 114 abgedeckten tatsächlichen Pixel des Zeilendetektors 110 mit dem anteiligen Pixelwert, mit welchem das betreffende Pixel (in Richtung der Detektorzeile gesehen) innerhalb der Breite des Vergleichspixels des Zeilendetektors 114 liegt.

Diese Vorgehensweise wird pixelweise für jedes Vergleichspixel des zu überwachenden Zeilendetektors 114 und das entsprechende Vergleichspixel des Referenz-Zeilendetektors 110 durchgeführt.

Anstelle der Wahl der tatsächlichen Pixel mit der größeren geometrischen Abmessung als Vergleichspixel können jedoch auch jede beliebige andere Breite einer Detektorzeile bzw. die durch diese Breite abgedeckten tatsächlichen Pixel als Vergleichspixel gewählt werden.

Wird bei einer derartigen Überwachung festgestellt, dass der zu überwachende Zeilendetektor keine korrekten Ergebnisse liefert, also die Vorrichtung 100 zur Röntgeninspektion von Produkten bzw. die Röntgenstrahlungsdetektorvorrichtung 108 ein "Nicht-Bereit-Signal" erzeugt, so kann eine übergeordnete Steuereinheit (nicht dargestellt), welcher das "Nicht-Bereit-Signal" zugeführt ist und welche zur Steuerung einer gesamten, die Vorrichtung 100 umfassenden Verarbeitungsanlage ausgebildet ist, die Verarbeitungsanlage stillsetzen und/oder ein Alarmsignal erzeugen.

Die übergeordnete Steuereinheit kann jedoch auch so ausgebildet sein, dass trotz Vorliegen eines "Nicht-Bereit-Signals" die Anlage weiter betrieben wird, wobei die Auswerte- und Steuereinheit 136 der Vorrichtung zur Röntgeninspektion 100 in einem solchen Notbetriebsmodus so weiterarbeitet, dass dabei nur der bzw. die korrekt arbeitenden Zeilendetektoren angesteuert und/oder die betreffenden Bilddatensignale für eine Auswertung verwendet werden. Ein

EP 3 734 259 B1

derartiger Notbetrieb kann beispielsweise so lange aufrechterhalten werden, bis eine bestimmte Produktcharge abgearbeitet wurde.

In diesem Notbetriebsmodus kann die Auswerte- und Steuereinheit 136 auch weitere Maßnahmen vorsehen, beispielsweise eine spezielle Ansteuerung der wenigstens einen Röntgenstrahlungsquelle 106. So kann zur Erhöhung der Detektorgenauigkeit des bzw. der verbleibenden Zeilendetektoren, insbesondere zur Verbesserung des Signal-Rausch-Verhältnisses beispielsweise die Leistung der Röntgenstrahlungsquelle 106 erhöht werden. Damit kann die teilweise vorhandene Redundanz von spektral auflösenden Zeilendetektoren und nicht spektral auflösenden Zeilendetektoren in einem Notbetriebsmodus genutzt werden, um die Vorrichtung zur Röntgeninspektion 100 mit dann allerdings reduzierten Fähigkeiten weiter zu betreiben. Reichen diese Fähigkeiten bzw. Eigenschaften jedoch aus, um, abhängig vom Anwendungsfall, eine ausreichende Inspektion der zu untersuchenden Produkte zu gewährleisten, so stellt der Notbetriebsmodus einen beträchtlichen Vorteil dar.

Im Folgenden wird erläutert, welche Informationen der spektral auflösende Sensor 114 liefern kann, die zu einer verbesserten Erkennungsgenauigkeit bei einer Verarbeitung der Bilddaten der höher ortsauflösenden nicht spektral auflösenden Sensoren 110, 112 führen.

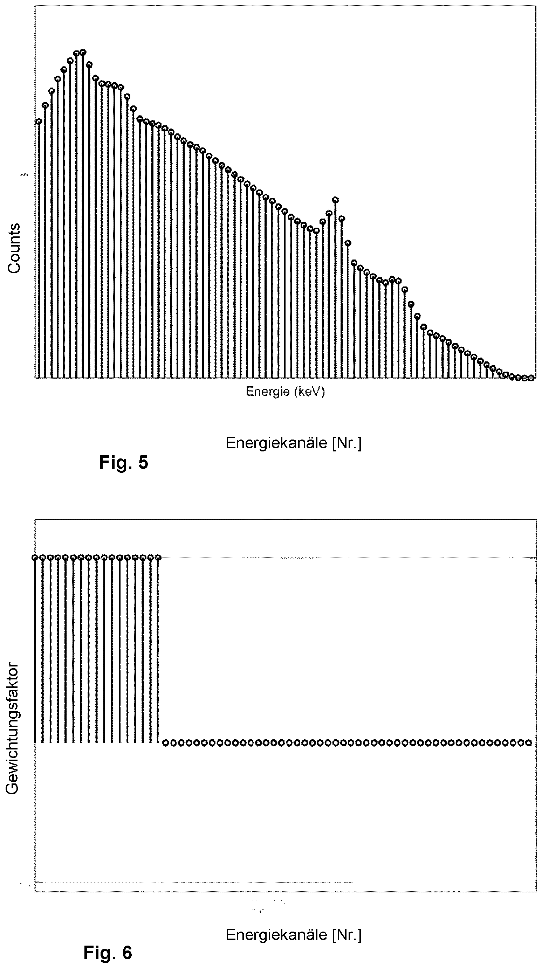

Fig. 5 zeigt beispielhaft die Bilddaten, die von dem spektral auflösenden Zeilendetektor 114 für ein bestimmtes Pixel geliefert werden. Hierbei ist der gesamte erfasste Energiebereich, beispielsweise von 20 keV bis 80 keV, in eine bestimmte Anzahl von Energiekanälen eingeteilt, wobei jeder Energiekanal eine bestimmte (üblicherweise konstante) spektrale Breite aufweist, die sich aus der Breite des gesamten erfassten spektralen Bereichs geteilt durch die Anzahl von Energiekanälen, beispielsweise 128 oder 256 Energiekanäle, ergibt. Der Zeilendetektor 114 liefert für jeden Energiekanal einen Spektralwert, der in Fig. 5 mit "Counts" bezeichnet ist, da ein derartiger spektral auflösender Zeilendetektor in der Regel einzelne Photonen zählt, und die registrierten Photonen, abhängig von deren Energie, einem bestimmten Energiekanal zuordnet. Der in Fig. 5 dargestellte Verlauf entspricht einem typischen Hellbild, welches von dem Zeilendetektor 114 ohne das Vorhandensein eines Produkts im Strahlengang des Röntgenstrahls 116 erzeugt wird.

Die Spektralwerte werden mit dem Bilddatensignal der Auswerte- und Steuereinheit 136 als Bilddaten übermittelt. Die Auswerte- und Steuereinheit 136 kann diese Bilddaten in vielfältiger Weise auswerten, um Informationen zu gewinnen, um diese Bilddaten entweder zu einer verbesserten Auswertung der Bilddaten der nicht spektral auflösenden Zeilendetektoren 110, 112 zu verwenden oder um die Bilddaten des spektral auflösenden Zeilendetektors 114 mit den Bilddaten der nicht spektral auflösenden Zeilendetektoren 110, 112 zu einem Gesamtbild zu kombinieren, welches, gegebenenfalls unter Verwendung von weiteren Informationen, die aus den Bilddaten des nicht spektral auflösenden Zeilendetektors 114 gewonnen werden, eine verbesserte Bildauswertung ermöglicht.

Die Fähigkeit einer spektralen Auflösung des Zeilendetektors 114 kann ausgenutzt werden, um Teilbilddaten zur Erzeugung eines Dual-Energy-Bildes zu gewinnen. Hierzu kann die Auswerte- und Steuereinheit 136 pixelweise eine beliebige Gewichtung der Spektralwerte vornehmen. Eine solche Gewichtung kann dadurch bewirkt werden, dass jedem einzelnen Energiekanal ein Faktor zugeordnet wird, der mit dem jeweiligen Spektralwert multipliziert wird. Hierdurch kann auch eine scharfe Beschränkung des Spektrums erreicht werden, wenn ausgewählten Energiekanälen der Faktor Null zugeordnet wird.

Fig. 6 zeigt ein Gewichtungsprofil, welches für jeden Energiekanal einen separaten Gewichtungsfaktor aufweist. Dieses Profil sieht für eine Anzahl von Energiekanälen am unteren Bereich des gesamten Spektrums eine konstante Gewichtung, beispielsweise mit dem Faktor eins, vor. Alle anderen Energiekanäle werden mit dem Faktor Null gewichtet. Die so gewichteten Spektralwerte können zur Erzeugung eines Summen-Spektralwerts addiert werden. Die Summen-Spektralwerte können dann als Teilbilddaten zur Erzeugung eines Dual-Energy-Bildes verwendet werden, wobei ein nicht spektral auflösender Zeilendetektor, beispielsweise der Zeilendetektor 110 bei der Ausführungsform gemäß Fig. 2, weitere Teilbilddaten zur Erzeugung des Dual-Energy-Bildes liefern kann. Die Auswerte- und Steuereinheit 136 kombiniert die Grauwert-Bilddaten, die der nicht spektral auflösende Zeilendetektor 110 liefert, mit den Teilbilddaten des spektral auflösenden Zeilendetektors 114 zu dem gewünschten Dual-Energy-Bild, wobei die Auswerte- und Steuereinheit 136 hierzu die jeweiligen Teilbilddaten pixelweise gewichtet. Diese Gewichtung kann für die von dem spektral auflösenden Zeilendetektor 114 gelieferten Bilddaten bereits im Rahmen der Gewichtung der Energiekanäle erfolgen.

Diese Vorgehensweise bietet den Vorteil, dass, anders als im Fall eines nicht spektral auflösenden Zeilendetektors, die Energiekanäle, die zur Bildung der Teilbilddaten für ein Dual-Energy-Bild ausgewählt werden, nicht nur mit einem konstanten Faktor, sondern mit einem variablen Faktor gewichtet werden können.

Wie bereits vorstehend erläutert, kann der spektral auflösende Zeilendetektor 114 auch so ausgebildet sein, dass dieser eine Vorauswahl trifft, welche Energiekanäle im Rahmen eines Bilddatensignals zur Auswerte- und Steuereinheit 136 übermittelt werden. Beispielsweise kann der Zeilendetektor 114 manuell oder von der Auswerte- und Steuereinheit 136 so eingestellt werden, dass er nur bestimmte Energiekanäle als Bilddatensignal ausgibt. Der Zeilendetektor 114 kann auch so ausgebildet sein, dass er die ausgewählten Energiekanäle bereits integriert ausgibt, d. h. die Spektralwerte der ausgewählten Energiekanäle aufsummiert.

In diesem Fall ergibt sich für die Auswerte- und Steuereinheit 136 ein geringerer Aufwand für die Verarbeitung der Bilddaten des Zeilendetektors 114.

Somit ermöglicht der nicht spektral auflösende Zeilendetektor 114 die Erzeugung eines Dual-Energy-Bildes unter Verwendung eines flexiblen Spektrums. Dieses kann durch die einfache Auswertung des Bilddatensignals des Zeilendetektors 114 festgelegt werden, oder der Zeilendetektor 114 wird so angesteuert, dass er bereits entsprechende spektral beschränkte Bilddaten bzw. sogar Summen-Spektralwerte liefert (siehe oben).

Auf diese Weise kann mittels eines einzigen nicht spektral auflösenden Zeilendetektors 110, der jedoch eine hohe Ortsauflösung aufweist, und eines spektral auflösenden Zeilendetektors 114, der allerdings nur eine relativ geringe Ortsauflösung aufweist, ein variables Dual-Energy-Bild erzeugt werden. Das Spektrum des spektral auflösenden Zeilendetektors 114 kann dabei so variiert werden, dass bestimmte Merkmale eines zu untersuchenden Produkts in dem Dual-Energy-Bild besser, beispielsweise mit einem höheren Kontrast, erkennbar sind.

Die Auswertung der mittels eines einzigen Scans gewonnenen Bilddaten der beiden Zeilendetektoren 110, 114 kann auch so erfolgen, dass mehrere Teilauswertungen durchgeführt werden. Beispielsweise können unterschiedliche Dual-Energy-Bilder unter Verwendung verschieden gewichteter Bilddaten des spektral auflösenden Zeilendetektors 114 erzeugt werden. Beispielsweise kann das Spektrum der Bilddaten des Zeilendetektors 114 in einer Auswertung so gewählt werden (beispielsweise durch eine entsprechende Gewichtung), dass Fremdkörper aus einem bestimmten Material, beispielsweise Stahl, mit hohem Kontrast erkennbar sind. In einer weiteren Auswertung kann das Spektrum der Bilddaten des Zeilendetektors 114 anders gewählt werden, beispielsweise um ein Dual-Energy-Bild zu erzeugen, in dem Fremdkörper aus einem anderen Material, beispielsweise Polyethylen, mit hohem Kontrast zu erkennen sind.

Neben der Erzeugung von Teilbilddaten für ein Dual-Energy-Bild bieten der eine oder die mehreren spektral auflösenden Zeilendetektoren noch weitere Vorteile. Insbesondere kann die Auswertung eines auf diese Weise erzeugten Dual-Energy-Bildes oder die Auswertung eines Dual-Energy-Bildes, welches ausschließlich unter Verwendung von Bilddaten, die mittels nicht spektral auflösender Sensoren erzeugt wurden, verbessert werden. Hierbei handelt es sich um Verbesserungen, die auf der Ebene der Bildauswertung greifen, d. h. bei der Analyse eines bereits erzeugten Gesamtbildes (bei einem derartigen Gesamtbild handelt es sich immer um ein Graustufenbild).

Die spektrale Auflösung, die ein solcher Zeilendetektor liefert, ermöglicht es, unter Anwendung geeigneter Auswerteverfahren Informationen zu gewinnen, aus welchem Material oder welchen Materialien das zu untersuchende Produkt besteht. Allerdings lassen sich diese Informationen nur mit der geringeren Ortsauflösung des spektral auflösenden Zeilendetektors pixelweise bestimmen. Weiterhin ist es möglich, aus den Bilddaten des wenigstens einen spektral auflösenden Zeilendetektors Informationen über die Dicke einer oder mehrerer Materialschichten zu gewinnen, aus denen das zu untersuchende Produkt besteht.

Informationen, aus welchem Material bzw. welchen Materialien das zu untersuchende Produkt besteht, können durch verschiedene Auswerteverfahren der spektralen Daten, die der spektral auflösende Zeilendetektor liefert, gewonnen werden. Beispielsweise ist es möglich, durch das Durchstrahlen eines Produkts, das aus einem bekannten Material bzw. einer bekannten Materialkombination besteht, den spektralen Verlauf der Dämpfung zu ermitteln, welches das Produkt verursacht. Hierzu kann zunächst ein Hellbild detektiert werden, d. h. der spektrale Verlauf der Röntgenstrahlung $I_0(E_k,x_i)$ ($I_0$ bezeichnet die ohne Produkt detektierte Strahlungsintensität; $E_k$ bezeichnet die Energie der Röntgenstrahlungsquanten im Energiekanal k; $x_i$ bezeichnet den Ort im Zeilendetektor, insbesondere das Pixel Nr. i), wenn kein Produkt durchstrahlt wird. Das Messergebnis $I(E_k,x_i)$ (I bezeichnet die mit Produkt detektierte Strahlungsintensität) bei Durchstrahlen eines Produkts liefert somit zusammen mit dem Hellbild jeweils die durch die Absorption im Produkt verursachte Dämpfung $D(E_k, x_i)$, wobei sich die Dämpfung aus dem Lambert-Beer'schen Gesetzt ergibt:

$$D(E_k,x_i) = \ln [ I(E_k,x_i) / I_0(E_k,x_i) ] = \mu(E_k) \bullet d_i \qquad \text{Gl. (1)}$$

wobei mit $d_i$ die Dicke des Produkts im Strahlengang des Strahlungsanteils bezeichnet ist, der vom Pixel $x_i$ detektiert wird und mit $\mu(E_k)$ der Absorptionskoeffizient des Materials im Strahlengang. Im Falle einer Materialkombination mit einer zumindest näherungsweise konstanten Zusammensetzung innerhalb des Strahlengangs kann ein mittlerer Absorptionskoeffizient für die betreffende Materialkombination verwendet werden. Da die Dicke $d_i$ nur einen konstanten Faktor (pro Pixel) darstellt, ergibt sich ein von der Dicke unabhängiger charakteristischer Verlauf Absorptionsverhaltens $\ln(I/I_0)$, das unter Verwendung des detektierten Spektrums (d. h. der Spektralwerte pro Pixel) und einem zuvor detektierten Spektrum eines Hellbildes bestimmt werden kann. Dieser spektrale Verlauf kann der Auswerte- und Steuereinheit für ein zu untersuchendes Produkt ohne Fremdkörper (im Folgenden auch als "Gutprodukt" bezeichnet) bekannt sein, d. h. entsprechende Informationen können in der Auswerte- und Steuereinheit gespeichert sein oder diese kann Zugriff auf entsprechende extern verfügbare Informationen haben.

Somit kann die Auswerte- und Steuereinheit durch einen Vergleich der gemessenen spektralen Intensitäten (Messung des Hellbildes und Messung des Produkts) mit einem einem oder mehreren bekannten spektralen Intensitätsverläufen für jeden Pixel feststellen, ob im betreffenden Strahlengang des Produkts ein bekanntes Material enthalten ist. Soll beispielsweise als Produkt Joghurt oder Käse untersucht werden, so kann die Auswerte- und Steuereinheit für jeden Pixel des spektral auflösenden Zeilendetektors bestätigen, ob der spektrale Verlauf mit dem erwarteten Produkt übereinstimmt. Weicht der gemessene spektrale Verlauf um mehr als eine zulässige Abweichung von dem bekannten Verlauf ab, so kann bereits anhand der Bilddaten des spektral auflösenden Sensors darauf geschlossen werden, dass in dem jeweiligen Pixel entsprechenden Teilvolumen des Produkts ein Fremdkörper enthalten ist.

Kann jedoch bestätigt werden, dass an dem betreffenden Pixel keine unzulässigen Abweichungen von dem zu erwartenden charakteristischen spektralen Verlauf vorliegen, kann von einem Gutprodukt in dem jeweiligen Strahlengang ausgegangen werden. Da der Absorptionskoeffizient $\mu(E_k)$ des betreffenden Materials ebenfalls bekannt ist und in der Auswerte- und Steuereinheit gespeichert oder dieser zumindest zugänglich gemacht sein kann, kann die betreffende Dicke des Produkts bzw. Länge des Strahlengangs durch das Produkt (bezogen auf den jeweiligen Pixel $x_i$) berechnet werden.

Mit dieser Information kann die Auswerte- und Steuereinheit die Auswertung eines Graubilds, das beispielsweise mittels des vorstehend erläuterten Dual-Energy-Verfahrens erzeugt wurde, unter Verwendung eines dynamischen Schwellenwerts vornehmen, der zur Erkennung von Fremdkörpern verwendet wird.

Bei der Röntgeninspektion von Produkten zum Auffinden von Fremdkörpern, d. h. jeglicher Art von zu detektierenden unerwünschten Bestandteilen oder Elementen eines zu untersuchenden Produkts, ist es üblich, einen statischen Schwellenwert zu verwenden. Weist beispielsweise ein Fremdkörper eine höhere Absorption auf als das ihn umgebende Material des zu untersuchenden Produkts, so wird man den statischen Schwellenwert in einen Bereich legen, der einerseits eine möglichst geringe Wahrscheinlichkeit eines Falschalarms erzeugt und andererseits eine ausreichend sichere Erkennung von Fremdkörpern ermöglicht. Beispielsweise kann hierzu aus dem Gesamtbild des Produkts ein mittlerer Grauwert ermittelt werden, der zur Ermittlung des statischen Schwellenwerts mit einem vorbekannten Faktor multipliziert wird.

Da das zu analysierende Graubild im Fall eines Produkts mit unbekannten Schwankungen in der Dicke oder in einem Fall, in dem mehrere Produkte, beispielsweise Fleischteile, einander überlappen können, größere Bereiche mit deutlich unterschiedlicher Helligkeit bzw. einem deutlich unterschiedlichen Grauwert aufweisen kann (die Absorption der Röntgenstrahlung durch das Produkt ist von der Materialdicke abhängig), führt die Verwendung eines derart statischen Schwellenwerts zu Problemen. Ist jedoch infolge der Information, die der spektral auflösende Zeilendetektor liefert, bekannt, dass auch in den dunkleren Bereichen nur "zulässiges" Material vorliegt, so kann die Auswerte- Steuereinheit davon ausgehen, dass zumindest die größeren dunkleren Bereiche keine Fremdkörper darstellen, sondern dass es sich um Dickenschwankungen des Produkts bzw. um überlappende Produktbereiche handelt. Somit kann die Auswerte- und Steuereinheit zur Analyse dieser Bereiche jeweils einen eigenen Schwellenwert festlegen. Dieser dynamische Schwellenwert kann von Bereich zu Bereich des zu analysierenden Graubildes variieren. Damit kann die Wahrscheinlichkeit eines Falschalarms reduziert werden.

Die Kombination wenigstens eines nicht spektral auflösenden Zeilendetektors mit einer höheren Ortsauflösung und wenigstens eines spektral auflösenden Zeilendetektors mit geringerer Ortsauflösung kann somit verwendet werden, um eine vorteilhafte Erzeugung eines Gesamtbildes, insbesondere eines Dual-Energy-Bildes oder Multiple-Energy-Bildes, aus den jeweiligen Bilddaten zu erreichen und/oder um eine vorteilhafte Auswertung eines derart erzeugten Dual-Energy-Bildes oder Multiple-Energy-Bildes oder eines in an sich bekannter Weise durch die Verwendung von Bilddaten von nicht spektral auflösenden Zeilendetektoren erzeugten Dual-Energy-Bildes oder Multiple-Energy-Bildes zu ermöglichen.

Bezugszeichenliste

| | |
|---|---|
| 100 | Vorrichtung zur Röntgeninspektion |
| 102 | Produkt |
| 104 | Strahlungserzeugungsvorrichtung |
| 106 | Röntgenstrahlungsquelle |
| 108 | Röntgenstrahlungsdetektorvorrichtung |
| 110 | nicht spektral auflösender Zeilendetektor |
| $110_k$ | Modul (Index k größer gleich 1) |
| 112 | nicht spektral auflösender Zeilendetektor |
| 114 | spektral auflösender Zeilendetektor |
| $114_i$ | Modul (Index i größer gleich 1) |
| 116 | fächerförmiger Röntgenstrahl |
| 118 | Pixelzeile |
| 120 | Pixelzeile |
| 122 | Pixelzeile |
| 124 | Träger |
| 126 | Träger |

128 Gehäuse
130 Öffnung
132 Auswerte- und Steuereinheit
134 Datenerfassungseinheit
136 Bildverarbeitungseinheit
138 Röntgenstrahlungsfilter
140 Stoßstelle (Module 110a, 110b, 114c)
142 Stoßstelle (Module 114a, 114b, 114c

200 Röntgenstrahlungsdetektorvorrichtung

**Patentansprüche**

1. Vorrichtung zur Röntgeninspektion von Produkten, insbesondere von Lebensmitteln, mit einer Röntgenstrahlungsdetektorvorrichtung (108), mit einer Auswerte- und Steuereinheit (132) und mit einer Strahlungserzeugungsvorrichtung (104) mit mindestens einer Röntgenstrahlungsquelle (106) zur Erzeugung von Röntgenstrahlung mit einer vorgegebenen spektralen Breite, wobei die Vorrichtung derart ausgebildet ist, dass die Röntgenstrahlung ein in einer Bewegungsrichtung mit einer vorgegebenen Bewegungsgeschwindigkeit bewegtes zu untersuchendes Produkt (102) durchstrahlt,

   (a) wobei die Röntgenstrahlungsdetektorvorrichtung (108) wenigstens einen ersten Zeilendetektor (110, 112) mit einer ersten diskreten Ortsauflösung aufweist, welcher zur Erfassung der Röntgenstrahlung entlang einer oder mehrerer paralleler erster Erfassungslinien quer zu einer Bewegungsrichtung einer Relativbewegung zwischen dem zu untersuchenden Produkt (102) und der Röntgenstrahlungsdetektorvorrichtung (108) ausgebildet ist und welcher derart ausgebildet ist, dass er die durch das zu untersuchende Produkt (102) hindurchgetretene Röntgenstrahlung über eine vorgegebene Erfassungsbreite nicht-spektral aufgelöst detektiert und erste Bilddaten erzeugt, wobei die ersten Bilddaten einen Grauwert für jeden diskreten Ort umfassen,
   (b) wobei die Röntgenstrahlungsdetektorvorrichtung (108) wenigstens einen zweiten Zeilendetektor (114) mit einer zweiten diskreten Ortsauflösung aufweist, die kleiner als die erste diskrete Ortsauflösung ist, wobei der wenigstens eine zweite Zeilendetektor (114) derart ausgebildet ist, dass er die durch das zu untersuchende Produkt (102) hindurchgetretene Röntgenstrahlung entlang einer oder mehrerer paralleler zweiter Erfassungslinien, welche parallel zu den ersten Erfassungslinien verlaufen, erfasst und über die vorgegebene Erfassungsbreite diskret spektral aufgelöst detektiert und zweite Bilddaten erzeugt, wobei die Röntgenstrahlungsquanten zur spektralen Auflösung, abhängig von deren Energie, einer vorgegebenen Anzahl von Energiekanälen zugeordnet werden, und wobei die zweiten Bilddaten für jeden diskreten Ort einen Spektralwert für jeden Energiekanal umfassen, und
   (c) wobei die Auswerte- und Steuereinheit (132), welcher die ersten und zweiten Bilddaten zugeführt sind, zur Auswertung der ersten und zweiten Bilddaten derart ausgebildet ist, dass wenigstens ein vorgegebenes Merkmal des zu untersuchenden Produkts (102) durch eine Kombination der in den ersten und zweiten Bilddaten enthaltenen Informationen mit mindestens der ersten diskreten Ortsauflösung detektiert wird.

2. Vorrichtung nach Anspruch 1, **dadurch gekennzeichnet, dass** das vorgegebene Merkmal des zu untersuchenden Produkts (102) wenigstens ein Merkmal aus der Gruppe ist: Position eines Fremdkörpers innerhalb des Produkts, Größe des Fremdkörpers, Material des Fremdkörpers, Dicke des Produkts, Material des Produkts, Dicke von Materialschichten des Produkts, Position von Überlappungsbereichen von Produkten (102).

3. Vorrichtung nach einem der vorhergehenden Ansprüche, **dadurch gekennzeichnet, dass** die Zeilendetektoren (110, 112; 114) auf einem gemeinsamen Träger (124, 126) und/oder in einem gemeinsamen Gehäuse (128) und/oder einem gemeinsamen Strahlenschutzgehäuse, vorgesehen sind.

4. Vorrichtung nach einem der Ansprüche 1 bis 3, **dadurch gekennzeichnet, dass** die Auswerte- und Steuereinheit (132) so ausgebildet ist, dass sie den wenigstens einen ersten und den wenigstens einen zweiten Zeilendetektor (110, 112; 114) derart ansteuert, dass eine synchrone Erfassung der Bilddaten an den ersten und zweiten Erfassungslinien abhängig von der Geschwindigkeit der Relativbewegung zwischen dem zu untersuchenden Produkt (102) und der Röntgenstrahlungsdetektorvorrichtung (108) erfolgt, um an den ersten und zweiten Erfassungslinien jeweils Zeilen-Bilddaten zu erhalten, die im Wesentlichen demselben Strahlengang durch das zu untersuchende Produkt (102) entsprechen.

**5.** Vorrichtung nach einem der Ansprüche 1 bis 3, **dadurch gekennzeichnet, dass** die Auswerte- und Steuereinheit (132) derart ausgebildet ist, dass sie den wenigstens einen ersten und -den wenigstens einen zweiten Zeilendetektor (110, 112; 114) derart ansteuert, dass eine asynchrone Datenerfassung an den ersten und zweiten Erfassungslinien erfolgt, wobei vorzugsweise an den ersten und zweiten Erfassungslinien jeweils Zeilen-Bilddaten mit einer Zeilen-Abtastrate erfasst werden, die größer ist als eine Pixel-Pitch des jeweiligen Zeilendetektors dividiert durch die Bewegungsgeschwindigkeit des zu untersuchenden Produkts (102).

**6.** Vorrichtung nach einem der vorhergehenden Ansprüche, **dadurch gekennzeichnet, dass** die Auswerte- und Steuereinheit (132) derart ausgebildet ist, dass sie aus den Bilddaten des wenigstens einen ersten Zeilendetektors (110, 112) und des wenigstens einen zweiten Zeilendetektors (114) ein Gesamtbild erstellt, insbesondere durch Interpolation und/oder geometrische Transformation der Bilddaten des wenigstens einen zweiten Zeilendetektors (114) und/oder der Bilddaten des wenigstens einen ersten Zeilendetektors (110, 112).

**7.** Vorrichtung nach einem der vorhergehenden Ansprüche, **dadurch gekennzeichnet, dass** der wenigstens eine erste Zeilendetektor (110, 112) und der wenigstens eine zweite Zeilendetektor (114) in der Richtung der betreffenden Erfassungslinie jeweils mehrere gleichartige, an Stoßstellen aneinandergereihte Module ($110_k$; $114_i$) umfasst, und dass die Stoßstellen (140) des wenigstens einen ersten Zeilendetektors (110, 112) und die Stoßstellen (142) des wenigstens einen zweiten Zeilendetektors (114) gegeneinander versetzt sind.

**8.** Vorrichtung nach einem der vorhergehenden Ansprüche, **dadurch gekennzeichnet, dass**

(a) die Auswerte- und Steuereinheit (132) derart ausgebildet ist, dass sie für jeden diskreten Ort die Spektralwerte aller Energiekanäle der Bilddaten des wenigstens einen zweiten Zeilendetektors (114) gewichtet und die gewichteten Spektralwerte zu einem Summen-Spektralwert addiert oder
(b) dass der wenigstens eine zweite Zeilendetektor (110, 112) derart ausgebildet ist, dass er für jeden diskreten Ort nur Bilddaten mit Spektralwerten von vorbestimmte Energiekanäle ausgibt, und dass die Auswerte- und Steuereinheit (132) derart ausgebildet ist, dass sie diese gewichtet und die gewichteten Spektralwerte zu einem Summen-Spektralwert addiert, oder
(c) dass der wenigstens eine zweite Zeilendetektor (114) derart ausgebildet ist, dass er für jeden diskreten Ort nur Bilddaten mit Spektralwerten von vorbestimmte Energiekanäle zur Bildung eines Summen-Spektralwertes addiert, und die Auswerte- und Steuereinheit (132) zur Gewichtung dieses Summen-Spektralwerts ausgebildet ist,

und dass die Auswerte- und Steuereinheit (132) so ausgebildet ist, dass sie aus den ersten Bilddaten des wenigstens einen ersten Zeilendetektors (110, 112) und den derart verarbeiteten zweiten Bilddaten des zweiten Zeilendetektors (114) Dual-Energy-Bilddaten oder Multiple-Energy-Bilddaten erzeugt.

**9.** Vorrichtung nach Anspruch 8, **dadurch gekennzeichnet, dass** in der Auswerte- und Steuereinheit (132) Informationen gespeichert sind oder die Auswerte- und Steuereinheit (132) auf Informationen Zugriff hat, welche Gewichtungen von Energiekanälen für das Detektieren eines oder mehrerer bestimmter vorgegebener Merkmale des zu untersuchenden Produkts (102) bevorzugt geeignet sind.

**10.** Vorrichtung nach Anspruch 8 oder 9, **dadurch gekennzeichnet, dass** die Auswerte- und Steuereinheit (132) derart ausgebildet ist, dass sie die Bilddaten des wenigstens einen ersten Zeilendetektors (110, 112) und die Bilddaten des wenigstens einen zweiten Zeilendetektors (114) für ein bestimmtes zu untersuchendes Produkt (102) mehrfach auswertet, insbesondere für das Detektieren verschiedener vorgegebener Merkmale, wobei für jede Auswertung jeweils Dual-Energy-Bilddaten-Bilddaten oder Multiple-Energy-Bilddaten unter Verwendung einer anderen Gewichtung der Spektralwerte der Energiekanäle der Bilddaten des zweiten Zeilendetektors erzeugt werden.

**11.** Vorrichtung nach einem der vorhergehenden Ansprüche, **dadurch gekennzeichnet, dass** die Auswerte- und Steuereinheit (132) so ausgebildet ist, dass sie die Bilddaten des wenigstens einen zweiten Zeilendetektors (114) zur Erkennung von Produktbereichen mit unterschiedlichen Schichtdicken und/oder Bereichen von überlappenden Produkten auswertet und derartige Bereiche in den Bilddaten des wenigstens einen ersten Zeilendetektors (110, 112) oder in kombinierten Bilddaten identifiziert, und dass die Auswerte- und Steuereinheit (13) so ausgebildet ist, dass sie zur Inspektion der so identifizierten Bereiche einen Schwellenwert verwendet, der in Abhängigkeit von den Grauwerten eines oder mehrerer dieser Bereiche gesetzt wird.

**12.** Vorrichtung nach einem der vorhergehenden Ansprüche, **dadurch gekennzeichnet, dass** die Auswerte- und Steuereinheit (132) so ausgebildet ist, dass sie zur Überwachung des Zustands eines ausgewählten Zeilendetektors (110, 112, 114) Bilddaten des zu überwachenden Zeilendetektors (110, 112, 114) und Bilddaten eines als Referenz-Zeilendetektor ausgewählten Zeilendetektors (110, 112, 114) ermittelt, vorzugweise jeweils ohne Vorhandensein eines Produkts (102), wobei die Auswerte- und Steuereinheit (132) vorzugsweise so ausgebildet ist, dass sie zur Ermittlung der Bilddaten des wenigstens einen zweiten Zeilendetektors (114) die Spektralwerte eines, mehrerer oder aller Energiekanäle aufsummiert, und dass die Auswerte- und Steuereinheit (132) so ausgebildet ist, dass sie die Bilddaten des zu überwachenden Zeilendetektors (110, 112, 114) mit Bilddaten des Referenz-Zeilendetektors (110, 112, 114) vergleicht und bei Feststellen unzulässiger Abweichungen ein "Nicht-Bereit-Signal" erzeugt.

13. Vorrichtung nach Anspruch 12, **dadurch gekennzeichnet, dass** die Auswerte- und Steuereinheit (132) so ausgebildet ist, dass sie die Referenzbilddaten pixelweise, unter Verwendung von einander entsprechenden Vergleichspixeln, mit den Bilddaten des zu überwachenden Zeilendetektors (110, 112, 114) vergleicht, wobei die Vergleichspixel so gewählt sind, dass zwei einander entsprechende Vergleichspixel dieselbe Breite auf den jeweiligen Erfassungslinien abdecken, und wobei der Grauwert eines Pixels, das nicht mit der gesamten Breite innerhalb der Breite des jeweiligen Vergleichspixels liegt, bei der Bestimmung des Grauwertes des betreffenden Vergleichspixels mit dem Verhältnis gewichtet wird, mit welchem das Pixel mit seiner Breite innerhalb der Breite des Vergleichspixels liegt.

14. Vorrichtung nach einem der vorhergehenden Ansprüche, **dadurch gekennzeichnet, dass** der wenigstens eine erste Zeilendetektor (110, 112) und der zweite Zeilendetektor (114) derart angeordnet sind, dass die eine oder mehreren zweiten Erfassungslinien des wenigstens einen zweiten Zeilendetektors (114) nicht im Schatten des wenigstens einen ersten Zeilendetektors liegen und die eine oder mehreren ersten Erfassungslinien des ersten Zeilendetektors (110, 112) nicht im Schatten des zweiten Zeilendetektors (114) liegen.

15. Vorrichtung nach Anspruch 14, **dadurch gekennzeichnet, dass** der wenigstens eine erste Zeilendetektor (110, 112) und der wenigstens eine zweite Zeilendetektor (114), in Strahlungsrichtung gesehen, überlappend angeordnet sind, wobei die eine oder mehreren ersten und zweiten Erfassungslinien einen unterschiedlichen Abstand von der wenigstens einen Röntgenstrahlungsquelle (106) aufweisen.

## Claims

1. Device for the X-ray inspection of products, in particular foodstuffs, comprising an X-ray detector device (108), comprising an evaluation and control unit (132), and comprising a radiation generation device (104) which has at least one X-ray source (106) for generating X-rays of a predetermined spectral width, wherein the device is designed such that the X-rays radiate through a product to be examined (102), the product being moved in a direction of movement at a predetermined speed of movement,

   (a) wherein the X-ray detector device (108) has at least one first line detector (110, 112) which has a first discrete spatial resolution, is designed to capture the X-rays along one or more parallel first capture lines transverse to a direction of movement of a relative movement between the product to be examined (102) and the X-ray detector device (108), and is designed such that it detects, in a non-spectrally resolved manner, the X-rays that have passed through the product to be examined (102) over a predetermined capture width and generates first image data, wherein the first image data comprise a gray value for each discrete location,
   (b) wherein the X-ray detector device (108) has at least one second line detector (114) which has a second discrete spatial resolution which is smaller than the first discrete spatial resolution, wherein the at least one second line detector (114) is designed such that it captures the X-ray radiation that has passed through the product to be examined (102) along one or more parallel second capture lines which run parallel to the first capture lines, and detects said radiation in a discrete spectrally resolved manner over the predetermined capture width and generates second image data, wherein the X-ray quanta, for spectral resolution, are assigned, depending on their energy, to a predetermined number of energy channels, and wherein the second image data for each discrete location comprise a spectral value for each energy channel, and
   (c) wherein the evaluation and control unit (132), to which the first and second image data are supplied, is designed to evaluate the first and second image data such that at least one predetermined feature of the product to be examined (102) is detected via a combination of the information contained in the first and second image data and at least the first discrete spatial resolution.

2. Device according to claim 1, **characterized in that** the predetermined feature of the product to be examined (102) is at least one feature from the group: position of a foreign body within the product, size of the foreign body, material of the foreign body, thickness of the product, material of the product, thickness of material layers of the product, position of overlapping regions of products (102).

3. Device according to any of the preceding claims, **characterized in that** the line detectors (110, 112; 114) are provided on a common carrier (124, 126) and/or in a common housing (128) and/or in a common radiation protection housing.

4. Device according to any of claims 1 to 3, **characterized in that** the evaluation and control unit (132) is designed such that it controls the at least one first and the at least one second line detector (110, 112; 114) such that a synchronous capturing of the image data at the first and second capture lines takes place depending on the speed of the relative movement between the product to be examined (102) and the X-ray detector device (108), in order to obtain, at each of the first and second capture lines, line image data which substantially correspond to the same radiation path through the product to be examined (102).

5. Device according to any of claims 1 to 3, **characterized in that** the evaluation and control unit (132) is designed such that it controls the at least one first and the at least one second line detector (110, 112; 114) such that asynchronous data capturing takes place on the first and second capture lines, wherein line image data is preferably captured on each of the first and second capture lines at a line sample rate which is greater than a pixel pitch of the relevant line detector divided by the speed of movement of the product to be examined (102).

6. Device according to any of the preceding claims, **characterized in that** the evaluation and control unit (132) is designed such that it creates an overall image from the image data of the at least one first line detector (110, 112) and the at least one second line detector (114), in particular by interpolating and/or geometrically transforming the image data of the at least one second line detector (114) and/or the image data of the at least one first line detector (110, 112).

7. Device according to any of the preceding claims, **characterized in that** the at least one first line detector (110, 112) and the at least one second line detector (114) each comprise, in the direction of the corresponding capture line, a plurality of similar modules ($110_k$; $114_i$) arranged in a row next to one another at joints, **and in that** the joints (140) of the at least one first line detector (110, 112) and the joints (142) of the at least one second line detector (114) are offset from one another.

8. Device according to any of the preceding claims, **characterized in that**

   (a) the evaluation and control unit (132) is designed such that it weights the spectral values of all of the energy channels of the image data of the at least one second line detector (114) for each discrete location and adds the weighted spectral values to form a total spectral value or
   (b) **in that** the at least one second line detector (110, 112) is designed such that it outputs only image data which have spectral values of predetermined energy channels for each discrete location, **and in that** the evaluation and control unit (132) is designed such that it weights them and adds the weighted spectral values to form a total spectral value, or
   (c) **in that** the at least one second line detector (114) is designed such that for each discrete location it only adds image data which have spectral values of predetermined energy channels to form a total spectral value, and the evaluation and control unit (132) is designed to weight this total spectral value,

   **and in that** the evaluation and control unit (132) is designed such that it generates dual-energy image data or multiple-energy image data from the first image data of the at least one first line detector (110, 112) and the thus processed second image data of the second line detector (114).

9. Device according to claim 8, **characterized in that** information is stored in the evaluation and control unit (132) or the evaluation and control unit (132) has access to information about which weightings of energy channels are preferably suitable for detecting one or more specific predetermined features of the product to be examined (102).

10. Device according to claim 8 or claim 9, **characterized in that** the evaluation and control unit (132) is designed such that it evaluates several times the image data of the at least one first line detector (110, 112) and the image data of the at least one second line detector (114) for a specific product to be examined (102), in particular for detecting various predetermined features, dual-energy image data or multiple-energy image data being generated for each evaluation in each case by using a different weighting of the spectral values of the energy channels of the image data of the second line detector.

**11.** Device according to any of the preceding claims, **characterized in that** the evaluation and control unit (132) is designed such that it evaluates the image data of the at least one second line detector (114) to identify product regions with different layer thicknesses and/or regions of overlapping products and identifies such regions in the image data of the at least one first line detector (110, 112) or in combined image data, **and in that** the evaluation and control unit (13) is designed such that it uses a threshold value for inspecting the regions thus identified, which threshold value is set depending on the gray values of one or more of these regions.

**12.** Device according to any of the preceding claims, **characterized in that** the evaluation and control unit (132) is designed such that, in order to monitor the state of a selected line detector (110, 112, 114), it determines image data of the line detector (110, 112, 114) to be monitored and image data of a line detector (110, 112, 114) selected as a reference line detector, preferably in each case without the presence of a product (102), the evaluation and control unit (132) preferably being designed such that, in order to determine the image data of the at least one second line detector (114), it totals the spectral values of one, a plurality of or all of the energy channels, **and in that** the evaluation and control unit (132) is designed such that it compares the image data of the line detector (110, 112, 114) to be monitored with image data of the reference line detector (110, 112, 114) and generates a "not ready signal" if inadmissible deviations are identified.

**13.** Device according to claim 12, **characterized in that** the evaluation and control unit (132) is designed such that it compares, pixel by pixel, by using mutually corresponding comparison pixels, the reference image data with the image data of the line detector (110, 112, 114) to be monitored, the comparison pixels being selected such that two mutually corresponding comparison pixels cover the same width on the respective capture lines, and the gray value of a pixel which does not lie with its entire width within the width of the relevant comparison pixel being weighted, when determining the gray value of the corresponding comparison pixel, using the ratio with which the pixel lies with its width within the width of the comparison pixel.

**14.** Device according to any of the preceding claims, **characterized in that** the at least one first line detector (110, 112) and the second line detector (114) are arranged such that the one or more second capture lines of the at least one second line detector (114) are not in the shadow of the at least one first line detector and the one or more first capture lines of the first line detector (110, 112) are not in the shadow of the second line detector (114).

**15.** Device according to claim 14, **characterized in that** the at least one first line detector (110, 112) and the at least one second line detector (114), viewed in the direction of radiation, are arranged in an overlapping manner, the one or more first and second capture lines being at different distances from the at least one X-ray source (106).

**Revendications**

**1.** Dispositif permettant l'inspection par rayons X de produits, en particulier de produits alimentaires, comportant un dispositif de détecteur de rayonnement X (108), comportant une unité d'évaluation et de commande (132) et comportant un dispositif de génération de rayonnement (104) comportant au moins une source de rayonnement X (106) permettant la production de rayonnement X d'une largeur spectrale prédéfinie, dans lequel le dispositif est réalisé de telle sorte que le rayonnement X traverse un produit (102) à examiner déplacé dans une direction de déplacement avec une vitesse de déplacement prédéfinie,

(a) dans lequel le dispositif de détecteur de rayonnement X (108) présente au moins un premier détecteur de ligne (110, 112) avec une première résolution spatiale discrète, lequel est réalisé pour la capture du rayonnement X le long d'une ou de plusieurs premières lignes de captage parallèles transversales à une direction de déplacement d'un déplacement relatif entre le produit (102) à examiner et le dispositif de détecteur de rayonnement X (108) et lequel est réalisé de telle sorte qu'il détecte le rayonnement X qui a traversé le produit (102) à examiner sur une largeur de captage prédéfinie avec une résolution non spectrale et qu'il produit des premières données d'image, dans lequel les premières données d'image comprennent une valeur de gris pour chaque emplacement discret, (b) dans lequel le dispositif de détecteur de rayonnement X (108) présente au moins un second détecteur de ligne (114) avec une seconde résolution spatiale discrète qui est inférieure à la première résolution spatiale discrète, dans lequel l'au moins un second détecteur de ligne (114) est réalisé de telle sorte qu'il capte le rayonnement X ayant traversé le produit (102) à examiner le long d'une ou de plusieurs secondes lignes de détection parallèles, qui s'étendent parallèlement aux premières lignes de détection, et les détecte avec une résolution spectrale discrète sur la largeur de captage prédéfinie et produit des secondes données d'image, dans lequel les quanta de rayonnement X sont affectés à un nombre prédéfini de canaux d'énergie pour la résolution spectrale, en fonction de leur énergie, et dans lequel les secondes données d'image comprennent pour chaque emplacement discret une valeur spectrale pour chaque canal d'énergie, et

(c) dans lequel l'unité d'évaluation et de commande (132), à laquelle sont amenées les premières et secondes données d'image, est réalisée pour l'évaluation des premières et secondes données d'image de telle sorte qu'au moins une caractéristique prédéfinie du produit (102) à examiner est détectée par une combinaison des informations contenues dans les premières et secondes données d'image avec au moins la première résolution spatiale discrète.

**2.** Dispositif selon la revendication 1, **caractérisé en ce que** la caractéristique prédéfinie du produit (102) à examiner est au moins une caractéristique du groupe : position d'un corps étranger à l'intérieur du produit, taille du corps étranger, matériau du corps étranger, épaisseur du produit, matériau du produit, épaisseur des couches de matériau du produit, position de zones de chevauchement de produits (102).

**3.** Dispositif selon l'une des revendications précédentes, **caractérisé en ce que** les détecteurs de lignes (110, 112 ; 114) sont prévus sur un support commun (124, 126) et/ou dans un boîtier commun (128) et/ou un boîtier de protection contre les rayons commun.

**4.** Dispositif selon l'une des revendications 1 à 3, **caractérisé en ce que** l'unité d'évaluation et de commande (132) est réalisée de telle sorte qu'elle commande l'au moins un premier et l'au moins un second détecteur de ligne (110, 112 ; 114) de telle sorte qu'un captage synchrone des données d'image au niveau des premières et secondes lignes de captage est effectué en fonction de la vitesse du déplacement relatif entre le produit (102) à examiner et le dispositif de détecteur de rayonnement X (108), afin d'obtenir au niveau des premières et secondes lignes de captage respectivement des données d'image de ligne qui correspondent sensiblement au même trajet de rayons à travers le produit (102) à examiner.

**5.** Dispositif selon l'une des revendications 1 à 3, **caractérisé en ce que** l'unité d'évaluation et de commande (132) est réalisée de telle sorte qu'elle commande l'au moins un premier et l'au moins un second détecteur de ligne (110, 112 ; 114) de telle sorte qu'un captage de données asynchrone est effectué sur les premières et secondes lignes de captage, dans lequel des données d'image de ligne sont de préférence captées sur les premières et secondes lignes de captage respectivement avec un taux d'échantillonnage de ligne qui est supérieur à un pas de pixel du détecteur de ligne respectif divisé par la vitesse de déplacement du produit (102) à examiner.

**6.** Dispositif selon l'une des revendications précédentes, **caractérisé en ce que** l'unité d'évaluation et de commande (132) est réalisée de telle sorte qu'elle établit une image globale à partir des données d'image de l'au moins un premier détecteur de ligne (110, 112) et de l'au moins un second détecteur de ligne (114), en particulier par interpolation et/ou transformation géométrique des données d'image de l'au moins un second détecteur de ligne (114) et/ou des données d'image de l'au moins un premier détecteur de ligne (110, 112).

**7.** Dispositif selon l'une des revendications précédentes, **caractérisé en ce que** l'au moins un premier détecteur de ligne (110, 112) et l'au moins un second détecteur de ligne (114) comprennent respectivement, dans la direction de la ligne de captage concernée, plusieurs modules ($110_k$ ; $114_i$) de même type alignés en des points de jonction, **et en ce que** les points de jonction (140) de l'au moins un premier détecteur de ligne (110, 112) et les points de jonction (142) de l'au moins un second détecteur de ligne (114) sont décalés les uns par rapport aux autres.

**8.** Dispositif selon l'une des revendications précédentes, **caractérisé en ce que**

(a) l'unité d'évaluation et de commande (132) est réalisée de telle sorte que, pour chaque emplacement discret, elle pondère les valeurs spectrales de tous les canaux d'énergie des données d'image de l'au moins un second détecteur de ligne (114) et additionne les valeurs spectrales pondérées en une valeur spectrale totale ou

(b) **en ce que** l'au moins un second détecteur de ligne (110, 112) est réalisé de telle sorte que, pour chaque emplacement discret, il ne délivre que des données d'image avec des valeurs spectrales de canaux d'énergie prédéterminés, **et en ce que** l'unité d'évaluation et de commande (132) est réalisée de telle sorte qu'elle pondère celles-ci et additionne les valeurs spectrales pondérées en une valeur spectrale totale, ou

(c) **en ce que** l'au moins un second détecteur de ligne (114) est réalisé de telle sorte que, pour chaque emplacement discret, il n'additionne que des données d'image avec des valeurs spectrales de canaux d'énergie prédéterminés pour la formation d'une valeur spectrale totale, et l'unité d'évaluation et de commande (132) est réalisée pour la pondération de cette valeur spectrale totale,

**et en ce que** l'unité d'évaluation et de commande (132) est réalisée de telle sorte qu'elle produit des données d'image à double énergie ou des données d'image à énergie multiple à partir des premières données d'image de l'au moins un premier détecteur de ligne (110, 112) et des secondes données d'image ainsi traitées du second détecteur de ligne (114).

9. Dispositif selon la revendication 8, **caractérisé en ce que** des informations sont mémorisées dans l'unité d'évaluation et de commande (132) ou l'unité d'évaluation et de commande (132) a accès à des informations concernant les pondérations de canaux d'énergie qui conviennent de préférence pour la détection d'une ou de plusieurs caractéristiques prédéfinies déterminées du produit (102) à examiner.

10. Dispositif selon la revendication 8 ou 9, **caractérisé en ce que** l'unité d'évaluation et de commande (132) est réalisée de telle sorte qu'elle évalue plusieurs fois les données d'image de l'au moins un premier détecteur de ligne (110, 112) et les données d'image de l'au moins un second détecteur de ligne (114) pour un produit (102) à examiner déterminé, en particulier pour la détection de différentes caractéristiques prédéfinies, dans lequel des données d'image de données d'image à double énergie ou des données d'image à énergie multiple sont respectivement produites pour chaque évaluation à l'aide d'une pondération différente des valeurs spectrales des canaux d'énergie des données d'image du second détecteur de ligne.

11. Dispositif selon l'une des revendications précédentes,
**caractérisé en ce que** l'unité d'évaluation et de commande (132) est réalisée de telle sorte qu'elle évalue les données d'image de l'au moins un second détecteur de ligne (114) pour la reconnaissance de zones de produits ayant des épaisseurs de couche différentes et/ou de zones de produits se chevauchant et qu'elle évalue de telles zones dans les données d'image de l'au moins un premier détecteur de ligne (110, 112) ou dans des données d'image combinées, **et en ce que** l'unité d'évaluation et de commande (13) est réalisée de telle sorte que, pour l'inspection des zones ainsi identifiées, elle utilise une valeur seuil qui est fixée en fonction des valeurs de gris d'une ou de plusieurs de ces zones.

12. Dispositif selon l'une des revendications précédentes,
**caractérisé en ce que** l'unité d'évaluation et de commande (132) est réalisée de telle sorte que, pour la surveillance de l'état d'un détecteur de ligne (110, 112, 114) sélectionné, elle détermine des données d'image du détecteur de ligne (110, 112, 114) à surveiller et des données d'image d'un détecteur de ligne (110, 112, 114) sélectionné comme détecteur de ligne de référence, de préférence respectivement sans présence d'un produit (102), dans lequel l'unité d'évaluation et de commande (132) est de préférence réalisée de telle sorte qu'elle additionne les valeurs spectrales d'un, de plusieurs ou de tous les canaux d'énergie pour la détermination des données d'image de l'au moins un second détecteur de ligne (114), **et en ce que** l'unité d'évaluation et de commande (132) est réalisée de telle sorte qu'elle compare les données d'image du détecteur de ligne (110, 112, 114) à surveiller avec des données d'image du détecteur de ligne de référence (110, 112, 114) et qu'elle produit un « signal non prêt » en cas de constatation d'écarts inadmissibles.

13. Dispositif selon la revendication 12, **caractérisé en ce que** l'unité d'évaluation et de commande (132) est réalisée de telle sorte qu'elle compare les données d'image de référence pixel par pixel, à l'aide de pixels de comparaison se correspondant mutuellement, avec les données d'image du détecteur de ligne (110, 112, 114) à surveiller, dans lequel les pixels de comparaison sont choisis de telle sorte que deux pixels de comparaison se correspondant mutuellement couvrent la même largeur sur les lignes de captage respectives, et dans lequel la valeur de gris d'un pixel qui ne se trouve pas avec toute sa largeur à l'intérieur de la largeur du pixel de comparaison respectif est pondérée, lors de la détermination de la valeur de gris du pixel de comparaison concerné, par le rapport avec lequel le pixel se trouve avec sa largeur à l'intérieur de la largeur du pixel de comparaison.

14. Dispositif selon l'une des revendications précédentes,
**caractérisé en ce que** l'au moins un premier détecteur de ligne (110, 112) et le second détecteur de ligne (114) sont disposés de telle sorte que les une ou plusieurs secondes lignes de captage de l'au moins un second détecteur de ligne (114) ne sont pas dans l'ombre de l'au moins un premier détecteur de ligne et les une ou plusieurs premières lignes de captage du premier détecteur de ligne (110, 112) ne sont pas dans l'ombre du second détecteur de ligne (114).

15. Dispositif selon la revendication 14, **caractérisé en ce que** l'au moins un premier détecteur de ligne (110, 112) et l'au moins un second détecteur de ligne (114) sont disposés en chevauchement, vus dans la direction de rayonnement, dans lequel les une ou plusieurs premières et secondes lignes de captage présentent une distance différente par rapport à l'au moins une source de rayonnement X (106).

**Fig. 1**

**Fig. 2a**

**Fig. 2b**

122 114$_{i-1}$ 142 114$_i$ 142 114$_{i+1}$

118

110$_{k-1}$ 140 110$_k$ 140 110$_{k+1}$

**Fig. 3**

122

118

**Fig. 4**

Fig. 5

Fig. 6

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20130079918 A1

- WO 2017205914 A1